Europäisches Patentamt

European Patent Office

Office européen des brevets

(11) Veröffentlichungsnummer: **0 351 575**
**A2**

(12) **EUROPÄISCHE PATENTANMELDUNG**

(21) Anmeldenummer: 89111362.3

(22) Anmeldetag: 22.06.89

(51) Int. Cl.⁴: **C12N 15/53 , C12N 15/74 ,**
**C12P 21/02 , C12N 9/04 ,**
**C12P 7/42 , C12P 7/40 ,**
**//(C12N9/04,C12R1:19)**

Die Bezeichnung der Erfindung wurde geändert (Richtlinien für die Prüfung im EPA, A-III, 7.3).

(30) Priorität: 21.07.88 DE 3824871

(43) Veröffentlichungstag der Anmeldung:
24.01.90 Patentblatt 90/04

(84) Benannte Vertragsstaaten:
CH DE FR GB IT LI NL SE

(71) Anmelder: **Gesellschaft für**
**Biotechnologische Forschung mbH (GBF)**
**Mascheroder Weg 1**
**D-3300 Braunschweig-Stöckheim(DE)**

(72) Erfinder: **Lerch, Hans-Phillip**
**Mascheroder Weg 1**
**D-3300 Braunschweig(DE)**
Erfinder: **Collins, John**
**Mascheroder Weg 1**
**D-3300 Braunschweig(DE)**
Erfinder: **Blöcker, Helmut**
**Mascheroder Weg 1**
**D-3300 Braunschweig(DE)**
Erfinder: **Hoppe, Jürgen**
**Mascheroder Weg 1**
**D-3300 Braunschweig(DE)**
Erfinder: **Kalwass, Helmut**
**Mascheroder Weg 1**
**D-3300 Braunschweig(DE)**
Erfinder: **Tsai, Hsin**
**Mascheroder Weg 1**
**D-3300 Braunschweig(DE)**

(74) Vertreter: **Boeters, Hans Dietrich, Dr. et al**
**Boeters, Bauer & Partner**
**Thomas-Wimmer-Ring 14**
**D-8000 München 22(DE)**

(54) D-HicDH-Gen, Herstellung von D-HicDH sowie Verfahren und dessen Verwendung.

(57) Die Erfindung betrifft das D-HicDH-Gen, DNA-Teilsequenzen des D-HicDH-Gens (insbesondere zur Verwendung als Translationsinitiator und/oder als Transkriptions-Terminator für die Herstellung von Genprodukten), Expressionsvektoren mit dem D-HicDH-Gen oder den DNA-Teilsequenzen, E.-coli-Stämme für die Herstellung von D-HicDH, das Enzym D-HicDH sowie Verfahren unter dessen Verwendung zur Überführung von Hydroxysäuren in Ketosäuren und umgekehrt.

Fig. 1

**D-HicDH-Gen, DNA-Teilsequenzen des Gens, Expressionsvektoren mit dem Gen oder den Teilsequenzen, E. coli-Stämme für die Herstellung von D-HicDH, D-HicDH sowie Verfahren unter dessen Verwendung**

D-2-Hydroxyisocapronsäure-Dehydrogenasen (D-HicDH) wurden zuerst von Hummel et al. (1984, 1985) aus verschiedenen gram-positiven Organismen einschließlich aus Lactobacillus casei ssp. pseudoplantarum (DSM 20008) isoliert. Dieses NAD (H) -abhängige Enzym katalysiert die stereospezifische und reversible Reduktion verschiedener aliphatischer und aromatischer 2-Ketocarbonsäuren, wobei die entsprechenden D-2-Hydroxycarbonsäuren gebildet werden. Es kann in industriellem Maßstab in Kombination mit L-HicDH, Schütte et al. (1984), und L-Leucin-Dehydrogenase (LeuDH), Schütte et. al. (1985), verwendet werden, um unter Einsatz von Ammoniak, polyethylenglycol-gebundenem NAD-Cofaktor und einer racemischen Mischung von 2-Hydroxycarbonsäuren in einem Membranreaktor für kontinuierliche Verfahren (Wandrey et al., 1984) L-Aminosäuren herzustellen. Das Enzym ist kristallisiert (Kallwaß et al., 1987) und in Bezug auf Teilsequenzen und immunologische Verfahren mit L-HicDH (Tsai et al., 1987) und einer Anzahl von anderen Amino- und Hydroxysäure-Dehydrogenasen verglichen worden, mit denen es nicht strukturverwandt ist (H.P. Lerch, unveröffentlichte Ergebnisse). Um ausreichende Mengen an Enzym für Umsetzungen in industriellem Maßstab zu gewinnen (anfänglich lagen die Ausbeuten nur bei 0,6 mg pro Liter Kulturflüssigkeit = 108 Einheiten (U)), und um einen ersten Schritt in Richtung eines gentechnologischen Ansatzes des Designs von Proteinen zu machen, das auf eine Änderung der Substratspezifitäten zielt, müssen alle Gene für diese Enzyme (D-HicDH, L-HicDH und L-LeuDH) kloniert werden. Es war die Aufgabe dieser Erfindung, das D-HicDH-Gen zu klonieren und zu charakterisieren und es in E. coli zu exprimieren.

Demzufolge wurde D-2-Hydroxyisocapronsäure-Dehydrogenase (D-HicDH) aus Lactobacillus casei gereinigt und teilweise sequenziert. Eines 65-mer-Oligonucleotid-Sonde, die den 23 N-terminalen Aminosäuren entspricht, wurde synthetisiert, und es wurde von der Genom-Region, die am stärksten hybridisierte, eine physikalische Karte angefertigt. Ein stark hybridisierendes Restriktionsfragment wurde hoch gereinigt und schließlich in pBR322 mit niederer Frequenz kloniert. Die ursprünglichen Klone produzierten D-HicDH spontan in einem Anteil von etwa 0,02 % des gesamten Proteins und zeigten Probleme bei der Lebensfähigkeit, da Wachstumsverzögerungsperioden von 10 bis 12 Stunden auftraten, wenn die stationären Kulturen in frischem Medium verdünnt wurden. Die Subklonierung in pGEM3-Plasmide für eine Sequenzierung unter gleichzeitiger Exo-III-Deletion führt zu Klonen, die die Charakteristika der Wachtumsinhibition nicht mehr zeigten, sondern nun D-HicDH in einer Menge von 3 bis 5 % des gesamten Proteins produzierten. Das Subklonieren strangabwärts eines doppelten $P_L P_R$ Promotors im Plasmid pJLA601 lieferte einen hoch induzierbaren Klon, der große Einschlußkörper aus weitgehend denaturierter D-HicDH bildet. Die Codon-Verwendung korrespondierte mit der anderer Lactobacillus-Gene, die kloniert worden sind. Es ist eine typische Shine-Dalgarno-Sequenz vorhanden, und eine Schlaufenstruktur am 3′-Ende des Gens stellt eine starke Terminator-Sequenz dar. Das Gen kodiert für eine Protein-Untereinheit von 38 kDa, wobei 65 % der Sequenz durch Vergleichen mit Peptid-Teilsequenzen des originalen Enzyms auf Korrelation geprüft werden konnten.

Gemäß einer Ausführungsform betrifft die Erfindung das D-HicDH-Gen, nämlich die Positionen 364 bis 1362 oder bis 1365 oder bis 1368 oder bis 1371 oder bis 1374 oder bis 1377 der folgenden DNA-Sequenz:

```
301    ATGCTTTTTCACAGAGGCATCTTGTATACGGTGAGTGGACAAATTGGAAAGGAAGTTTAA

              M  K  I  I  A  Y  G  A  R  V  D  E  I  Q  Y -F  K  Q  W
361    CACATGAAGATTATTGCTTACGGTGCTCGCGTTGACGAGATTCAATATTTCAAGCAATGG

           A  K  D  T  G  N  T  L  E  Y  H  T  E  F  L  D  E  N  T  V
421    GCCAAGGATACAGGCAACACACTTGAATACCATACAGAATTTCTCGATGAAAACACCGTT

           E  W  A  K  G  F  D  G  I  N  S  L  Q  T  T  P  Y  A  A  G
481    GAATGGGCTAAAGGGTTTGATGGCATCAATTCATTGCAGACAACGCCATATGCAGCCGGC

           V  F  E  K  M  H  A  Y  G  I  K  F  L .T  I  R  N  V  G  T
541    GTTTTTGAAAAAATGCACGCGTATGGTATCAAGTTCTTGACGATTCGGAATGTGGGTACG

           D  N  I  D  M  T  A  M  K  Q  Y  G  I  R  L  S  N  V  P  A
601    GATAACATTGATATGACTGCCATGAAGCAATACGGCATTCGTTTGAGCAATGTACCGGCT

           Y  S  P  A  A  I  A  E  F  A  L  T  D  T  L  Y  L  L  R  N
661    TATTCGCCAGCAGCGATTGCTGAATTTGCTTTGACCGATACTTTGTACTTGCTACGTAAT

           M  G  K  V  Q  A  Q  L  Q  A  G  D  Y  E  K  A  G  T  F  I
721    ATGGGTAAAGTACAGGCGCAACTACAGGCGGGCGATTATGAAAAAGCGGGCACCTTCATC

           G  K  E  L  G  Q  Q  T  V  G  V  M  G  T  G  H  I  G  Q  V
781    GGTAAGGAACTCGGTCAGCAAACCGTTGGCGTGATGGGCACCGGTCATATTGGACAGGTT

           A  I  K  L  F  K  G  F  G  A  K  V  I  A  Y  D  P  Y  P  M
841    GCTATCAAACTGTTCAAAGGCTTTGGCGCCAAAGTGATTGCTTACGATCCTTATCCAATG

           K  G  D  H  P  D  F  D  Y  V  S  L  E  D  L  F  K  Q  S  D
901    AAGGGCGATCACCCAGATTTTGACTATGTCAGCCTTGAAGACCTCTTTAAGCAAAGTGAT

           V  I  D  L  H  V  P  G  I  E  Q  N  T  H  I  I  N  E  A  A
961    GTCATTGATCTTCATGTTCCTGGGATTGAACAAAATACCCACATTATCAATGAAGCGGCA

           F  N  L  M  K  P  G  A  I  V  I  N  T  A  R  P  N  L  I  D
1021   TTTAATTTGATGAAACCGGGTGCGATTGTGATCAACACGGCTCGGCCAAATCTGATTGAC

           T  Q  A  M  L  S  N  L  K  S  G  K  L  A  G  V  G  I  D  T
1081   ACGCAAGCCATGCTCAGCAATCTTAAGTCTGGCAAGTTGGCCGGTGTCGGGATTGACACC

           Y  E  Y  E  T  E  D  L  L  N  L  A  K  H  G  S  F  K  D  P
1141   TATGAATACGAAACCGAGGACTTGTTGAATCTCGCCAAGCACGGCAGCTTCAAGGATCCG

           L  W  D  E  L  L  G  M  P  N  V  V  L  S  P  H  I  A  Y  Y
1201   TTGTGGGACGAGCTGTTGGGGATGCCAAATGTTGTCCTCAGCCCGCACATTGCCTACTAC

           T  E  T  A  V  H  N  M  V  Y  F  S  L  Q  H  L  V  D  F  L
1261   ACCGAGACGGCTGTGCATAATATGGTTTACTTCTCACTACAACATCTCGTTGATTTCTTG

           T  K  F  K  P  A  R  K  L  L  V  Q  Q  V  V  N  *
1321   ACCAAATTCAAACCAGCACGGAAGTTACTGGTCCAGCAAGTAGTCAACTGAATAGCAAAA
```

wobei das Gen an beliebiger Position um 1, 2 oder 3 Basentriplets verkürzt oder verlängert sein kann oder wobei ein Basentriplet des Gens durch ein anderes Basentriplet ersetzt sein kann oder wobei ein oder mehrere Basentriplets durch ein dieselbe Aminosäure kodierendes Basentriplet ersetzt sein können. Mit Hilfe der vorstehend angegebenen vollständig aufgeklärten Struktur des D-HicDH-Gens ist es erstmals möglich, gentechnologisch D-HicDH herzustellen.

Gemäß einer weiteren Ausführungsform betrifft die Erfindung eine DNA-Teilsequenz des vorstehend beschriebenen D-HicDH-Gens, nämlich die Positionen 343, 344, 345 oder 346 bis jeweils 363, wobei diese DNA-Teilsequenz an beliebiger Position um 1, 2 oder 3 Basen verkürzt oder verlängert sein kann oder wobei 1, 2 oder 3 Basen der DNA-Teilsequenz durch jeweils eine andere Base ersetzt sein können. Die genannte DNA-Teilsequenz kann man zur Translationsinitiation für die Herstellung von Genprodukten in gram-positiven oder gram-negativen Mikroorganismen verwenden, beispielsweise in E. coli.

Gemäß einer weiteren Ausführungsform betrifft die Erfindung eine DNA-Teilsequenz des vorstehend beschriebenen D-HicDH-Gens, nämlich 51 bis 93 Basen aus dem Positionsbereich von 1363 bis maximal 1455, wobei die DNA-Teilsequenz an beliebiger Position um 1, 2 oder 3 Basen verkürzt oder verlängert sein kann oder wobei 1, 2 oder 3 Basen der DNA-Teilsequenz durch jeweils eine andere Base ersetzt sein können, wobei jedoch der Positionsbereich von 1376 bis 1427 stets eingeschlossen ist. Diese DNA-Teilsequenz läßt sich als Transkriptions-Terminator für die Herstellung von Genprodukten in gram-positiven oder gram-negativen Mikroorganismen verwenden, beispielsweise in E. coli. Beispielsweise kann die DNA-Teilsequenz am 3'-Terminus des Gens des gewünschten Genprodukts angehängt verwendet werden, insbesondere als einziger Transkriptions-Terminator.

Gemäß einer weiteren Ausführungsform umfaßt die Erfindung Expressionsvektoren zur Expression von gewünschten Genprodukten in gram-positiven oder gram-negativen Mikroorganismen, beispielsweise in E. coli, wobei diese Expressionsvektoren eine vorstehend beschriebene DNA-Teilsequenz zur Translationsinitiation und/oder eine vorstehend beschriebene DNA-Teilsequenz als Transkriptions-Terminator umfassen.

Gemäß einer weiteren Ausführungsform umfaßt die Erfindung Expressionsvektoren zur Expression von D-HicDH in E. coli, wobei diese Vektoren das D-HicDH-Gen umfassen, das vorstehend beschrieben ist.

Gemäß einer weiteren Ausführungsform umfaßt die Erfindung E.-coli-Stämme für die Herstellung von D-HicDH mit Hilfe eines das vorstehend beschriebene D-HicDH-Gen umfassenden Expressionsvektors. Ein Beispiel ist der E.-coli-Stamm DSM 4535, der unter dieser Bezeichnung bei der Deutschen Sammlung von Mikroorganismen hinterlegt worden ist. D-HicDH wird in E. coli undegradiert im Zytoplasma produziert, wobei sich Ausbeuten von beispielsweise 8, 10, 25, 50 oder selbst noch mehr Prozent des Gesamtproteins erzielen lassen.

Gemäß einer weiteren Ausführungsform betrifft die Erfindung das Enzym D-HicDH der folgenden Aminosäuresequenz:

```
        M  K  I  I  A  Y  G  A  R  V  D  E  I  Q  Y  F  K  Q  W
361   CACATGAAGATTATTGCTTACGGTGCTCGCGTTGACGAGATTCAATATTTCAAGCAATGG

        A  K  D  T  G  N  T  L  E  Y  H  T  E  F  L  D  E  N  T  V
421   GCCAAGGATACAGGCAACACACTTGAATACCATACAGAATTTCTCGATGAAAACACCGTT

        E  W  A  K  G  F  D  G  I  N  S  L  Q  T  T  P  Y  A  A  G
481   GAATGGGCTAAAGGGTTTGATGGCATCAATTCATTGCAGACAACGCCATATGCAGCCGGC

        V  F  E  K  M  H  A  Y  G  I  K  F  L  T  I  R  N  V  G  T
541   GTTTTTGAAAAAATGCACGCGTATGGTATCAAGTTCTTGACGATTCGGAATGTGGGTACG

        D  N  I  D  M  T  A  M  K  Q  Y  G  I  R  L  S  N  V  P  A
601   GATAACATTGATATGACTGCCATGAAGCAATACGGCATTCGTTTGAGCAATGTACCGGCT

        Y  S  P  A  A  I  A  E  F  A  L  T  D  T  L  Y  L  L  R  N
661   TATTCGCCAGCAGCGATTGCTGAATTTGCTTTGACCGATACTTTGTACTTGCTACGTAAT

        M  G  K  V  Q  A  Q  L  Q  A  G  D  Y  E  K  A  G  T  F  I
721   ATGGGTAAAGTACAGGCGCAACTACAGGCGGGCGATTATGAAAAAGCGGGCACCTTCATC
```

```
            G   K   E   L   G   Q   Q   T   V   G   V   M   G   T   G   H   I   G   Q   V
    781     GGTAAGGAACTCGGTCAGCAAACCGTTGGCGTGATGGGCACCGGTCATATTGGACAGGTT

            A   I   K   L   F   K   G   F   G   A   K   V   I   A   Y   D   P   Y   P   M
    841     GCTATCAAACTGTTCAAAGGCTTTGGCGCCAAAGTGATTGCTTACGATCCTTATCCAATG

            K   G   D   H   P   D   F   D   Y   V   S   L   E   D   L   F   K   Q   S   D
    901     AAGGGCGATCACCCAGATTTTGACTATGTCAGCCTTGAAGACCTCTTTAAGCAAAGTGAT

            V   I   D   L   H   V   P   G   I   E   Q   N   T   H   I   I   N   E   A   A
    961     GTCATTGATCTTCATGTTCCTGGGATTGAACAAAATACCCACATTATCAATGAAGCGGCA

            F   N   L   M   K   P   G   A   I   V   I   N   T   A   R   P   N   L   I   D
    1021    TTTAATTTGATGAAACCGGGTGCGATTGTGATCAACACGGCTCGGCCAAATCTGATTGAC

            T   Q   A   M   L   S   N   L   K   S   G   K   L   A   G   V   G   I   D   T
    1081    ACGCAAGCCATGCTCAGCAATCTTAAGTCTGGCAAGTTGGCCGGTGTCGGGATTGACACC

            Y   E   Y   E   T   E   D   L   L   N   L   A   K   H   G   S   F   K   D   P
    1141    TATGAATACGAAACCGAGGACTTGTTGAATCTCGCCAAGCACGGCAGCTTCAAGGATCCG

            L   W   D   E   L   L   G   M   P   N   V   V   L   S   P   H   I   A   Y   Y
    1201    TTGTGGGACGAGCTGTTGGGGATGCCAAATGTTGTCCTCAGCCCGCACATTGCCTACTAC

            T   E   T   A   V   H   N   M   V   Y   F   S   L   Q   H   L   V   D   F   L
    1261    ACCGAGACGGCTGTGCATAATATGGTTTACTTCTCACTACAACATCTCGTTGATTTCTTG

            T   K   F   K   P   A   R   K   L   L   V   Q   Q   V   V   N   *
    1321    ACCAAATTCAAACCAGCACGGAAGTTACTGGTCCAGCAAGTAGTCAACTGAATAGCAAAA
```

wobei die folgenden Buchstaben bedeuten:

A = Alanin

D = Asparaginsäure

E = Glutaminsäure.

F = Phenylalanin

G = Glycin

H = Histidin

I = Isoleucin

K = Lysin

L = Leucin

M = Methionin

N = Asparagin

P = Prolin

Q = Glutamin

R = Arginin

S = Serin

T = Threonin

V = Valin

W = Tryptophan

Y = Tyrosin wobei das Enzym in beliebiger Position um 1, 2 oder 3 Aminosäuren verkürzt oder verlängert sein kann oder wobei eine Aminosäure durch eine andere Aminosäure ersetzt sein kann.

Schließlich betrifft die Erfindung gemäß einer Ausführungsform ein Verfahren zur Überführung von Hydroxysäuren in Ketosäuren oder von Ketosäuren in Hydroxysäuren, beispielsweise D-Hydroxysäuren, unter Verwendung von D-HicDH, insbesondere wenn das Enzym mit Hilfe eines erfindungsgemäßen E.-coli-Stamms erhalten worden ist.

MATERIALIEN

Restriktions-Endonukleasen, T4-DNA-Ligasen, T4-Polynukleotid-Kinase, Exonuklease III und Mungobohnen-Nuklease wurden von Boehringer Mannheim (Mannheim, BRD), Pharmacia (Freiburg, BRD), New England Biolabs (Schwalbach, BRD) oder GIBCO BRL (Eggenstein, BRD) bezogen. Sequenase stammte von United States Biochemicals (Cleveland, Ohio, USA), Lysozym von Serva (Heidelberg, BRD), Mutanolysin von Sigma (Deisenhofen, BRD) und Proteinase K von Merck (Darmstadt, BRD), /$\gamma$-$^{32}$P/ATP und '$\alpha$-$^{35}$S/dATP wurden von Amersham Buchler (Braunschweig, BRD) bezogen. Trypton, Pepton, Hefeextrakt und Casaminosäuren stammten von Difco (Detroit, Michigan, USA), Fleischextrakt von GIBCO BRL (Eggenstein, BRD). Das FPLC-System mit Mono-Q-Säule, Hydroxylapatit und DEAE-Sephacell stammte von Pharmacia (Freiburg, BRD). Das 65-mer-Oligonukleotid wurde auf dem Gen-Assembler von Pharmacia nach den Empfehlungen des Herstellers synthetisiert. Kürzere Oligonukleotide, die als Primer für die Sequenzierung verwendet wurden, wurden nach Frank et al. (1988) synthetisiert.

## BAKTERIENSTÄMME UND PLASMIDE

Chromosomale DNA wurde aus Lactobacillus casei ssp. pseudoplantarum DSM 20008 isoliert. Rekombinante Plasmide wurden in E. coli DH1 (F$^-$, end A1, hsd R17 (r$_k^-$, m$_k^+$) supE44, thi-1, $\lambda^-$, recA1, gyrA96, relA1) (Hanahan, 1983) transformiert. Das Plasmid pBR322 (Bolivar et al., 1977) und der Expressionsvektor pJLA 601 (Schauder et al., 1987) sind beschrieben. pGEM-Plasmide und pGEM-Sequenzierungsprimer stammten von Promega Biotec.. L. casei wurde in MRS-Medium (de Man et al., 1960) gezüchtet. Rekombinante E. coli-Stämme wurden auf SR Medium plattiert und darin vermehrt, um mögliche toxische Wirkungen der klonierten Lactobacillus-Gene zu verhindern. SR-Medium (1 l) wurde hergestellt, indem man die getrennt autoklavierten Lösungen A (100 ml) und B (900 ml) vereinigte. Lösung A besteht aus 170 mM KH$_2$PO$_4$, 720 mM K$_2$HPO$_4$; Lösung B enthält 20 g Trypton, 20 g Hefeextrakt, 20 g Casaminosäuren, 10 g Fleischextrakt und 5 ml 87 %iges Glycerin. TE Puffer enthält 0,01 M TRIS-HCl (pH 8,0) und 0,1 mM EDTA.

## Methoden

### HERSTELLUNG CHROMOSOMALER DNA AUS L. CASEI

1 l MRS Medium, ergänzt durch 1% Glycin (Bae et al., 1985) und ohne Magnesiumsulfat, wurde mit 30 ml einer Übernachtkultur inokuliert. Man ließ die Zellen 5 Stunden bei 37°C bis zur spätlogarithmischen Phase (OD$_{550}$ = 1,6) auf einem Rotationsschüttler wachsen. Die Kultur wurde 10 min lang bei 6000 x g zentrifugiert. Nachdem das Pellet mit 180 ml 0,1 M EDTA pH 7,0 und 180 ml 20 mM Kaliumphosphat, 10 mM EDTA pH 6,8 gewaschen worden war, wurden die Zellen in 10 ml 0,1 M HEPES, 10 mM EDTA, 0,3 M Raffinose, 0,5 % Triton X-100 pH 6,9 resuspendiert. Die Zellen wurden durch Zusatz von 40 mg Lysozym und 2500 U Mutanolysin wie von Monsen et al. (1983) für Streptokokken beschrieben lysiert. Die Proteine wurden durch Proteinase K (24 mg) in Gegenwart von 0,5 % SDS bei 60°C in einem Gesamtvolumen von 70 ml verdaut, bis die Lyse vollständig war. Nach Zugabe von 70 g CsCl und 8 ml Ethidiumbromid-Lösung (5 mg/ml in H$_2$O) wurde die chromosomale DNA von den fünf kryptischen Plasmiden dieses Lactobacillus-Stamms durch isopyknische Zentrifugation in einem Beckman Ti 50,2 Rotor innerhalb von 48 Stunden bei 38000 Upm und 18°C abgetrennt. Die chromosomale DNA wurde mit einer Pipette mit weiter Bohrung gesammelt, und das Ethidiumbromid wurde mit Isopropanol, gesättigt mit TE Puffer und CsCl, extrahiert. Die DNA wurde ausgiebig gegen mehrmals ausgetauschten TE Puffer dialysiert.

### ERZEUGEN VON ANTISERUM GEGEN D-HICDH UND SYNTHESE EINER OLIGONUKLEOTID-SONDE ZUM SCREENEN

Die D-HicDH wurde mit geringen Ängerungen nach dem Verfahren von Hummel et al. (1985) gereinigt. Nach einer weiteren Reinigung auf einer FPLC Mono-Q-Säule, Chromatographie über DEAE Sephacell und Hydroxylapatit erhielt man homogenes Enzym. Ein Kaninchen wurde mit 0,6 mg Protein aus dieser Präparation, vermischt mit 1 ml vollständigem Freundschem Adjuvans, immunisiert; zwei Wochen später wurden weitere 0,2 mg, vermischt mit 1 ml vollständigem Freund'schem Adjuvans, injiziert. Titration der Antiseren und Tests mit Hilfe von Western-Blots wurden ausgeführt, wie bereits beschrieben (Tsai et al., 1987).

Die ersten 25 N-terminalen Aminosäuren von D-Hic-DH wurden mit Hilfe von Gasphasen-Sequenzierung sequenziert, wobei man 0,13 mg Protein einsetzte, das vor dem Sequenzieren mit Methanol/HCl versetzt wurde, um eine Formyl- oder Acetylgruppe von der N-terminalen Aminosäure abzuspalten, die die Sequenzierung inhibiert hätte. Ensprechend dieser Sequenz wurde eine 65-mer-Oligonukleotid-Sonde synthetisiert (siehe Fig. 5).

## DNA-SEQUENZANALYSE

Eine Reihe von Subklonen mit progressiver, unidirektionaler Deletion wurde nach dem Verfahren von Henikoff (1984) generiert. Die Sequenzanalyse (Sanger et al., 1977) wurde an 2 ug mit Alkali denaturierter, superverknäulter Plasmid-DNA (Chen und Seeburg, 1985) unter Verwendung von /$\alpha$-$^{35}$S/dATP und Seque-nase, einer modifizierten Bakteriophagen T7 DNA Polymerase, nach den Vorschriften des Herstellers durchgeführt. Proben aus den Sequenzierungs-Reaktionsgemischen ließ man auf einem keilförmigen (0,2 - 0,6 mm), 6 %igen Polyacrylamid-Gel laufen.

## ANDERE TECHNIKEN

Kolonien von E. coli wurden nach Birnboim und Doly (1979) auf ihren Plasmid-Gehalt untersucht. Die Elution von DNA-Fragmenten aus Agarosegelen wurde wie von Zassenhaus et al. (1982) unter Einsatz eines Elektrophorese-Probenkonzentrators von ISCO, Modell 1750, durchgeführt. Die DNA wurde auf Nitrocellulose-Filter in 20 x SSC, wie von Southern (1975) beschrieben, und auf Nylon-Filter in 0,4 N NaOH, wie von Khandjian (1987) beschrieben, überführt. Die Endmarkierung der Oligonukleotide mit /$\gamma$-$^{32}$P/ ATP und T4 Polynukleotid- Kinase und die Hybridisierung auf den Filtern wurde gemäß Woods (1984) ausgeführt. SDS-PAGE von Proteinen wurde wie von Laemmli (1970) beschrieben durchgeführt. Für die Überführung der Proteine auf die Nitrocellulosefilter und das Durchführen der Western-Blots wurde das Verfahren von Towbin et al (1979) eingesetzt. Die Aktivität der D-HicDH wurde wie bereits beschrieben (Hummel et al., 1985) bestimmt.

SDS-Polyacrylamidgel-Elektrophorese (SDS-PAGE) von Peptiden, die man bei der proteolytischen Verdauung von D-HicDH erhielt, wurde unter Verwendung des PHAST-Systems und 8-25 PHAST-Gradientengelen (Pharmacia) durchgeführt.

## Ergebnisse

Die proteolytische Verdauung von D-HicDH war am erfolgreichsten, wenn man Cyanogenbromid und Trypsin einsetzte. Beide Reagentien bauen D-HicDH mit hoher Spezifität vollständig zu kürzeren Peptiden ab. Alle identifizierten Cyanogenbromid-Peptide korrespondieren mit Sequenzpositionen, die auf Methionin folgen, während nur ein tryptisches Fragment nach Asp(236) anstelle von Arg(234) abgespalten wurde. Trypsin spaltete auch nicht an allen Lysyl- und Arginylresten, was für die Bestimmung der Proteinsequenz hilfreich war. Während der tryptischen Spaltung nativer D-HicDH bildeten sich zwei in wäßrigem Puffer unlösliche Rumpfpeptide mit Molekulargewichten von annähernd 2000 und 6000, bestimmt durch SDS-PAGE. Das kleinere tryptische Rumpfpeptid entspricht der bekannten N-terminalen Sequenz. Eine Sequenz von 54 Aminosäuren (Positionen 181-234) des größeren Rumpfpeptids wurde aufgeklärt. In diesen Peptiden trat die tryptische Spaltung an den Positionen Arg(9), Lys(196) und Lys(224) nicht auf. Versetzte man sie dagegen mit Endoproteinase Arg-C, so bildete sich eine komplexe Mischung von Peptiden im Molekulargewichtsbereich von 38000 (intakte D-HicDH- Untereinheit) bis 1000 (untere Erfassungsgrenze der SDS-PAGE- Analyse), was für eine langsame Hydrolysegeschwindigkeit mit der Folge von unvollständiger Spaltung an vielen oder allen möglichen Spaltstellen spricht. Endoproteinase Glu-C baute D-HicDH weder unter sauren noch unter basischen Reaktionsbedingungen ab (Houmard und Drapeau, 1972).

Die Reinigung von Peptiden, die man durch Spaltung an Arginylresten erhielt, wurde durch die geringe Löslichkeit der Peptide und ihrer Neigung zur Bildung gemischter Aggregate erschwert. Diese Problem wurde gelöst, indem man D-HicDH citraconylierte und die säureempfindlichen Schutzgruppen (Dixon und Perham, 1968) nach der tryptischen Verdauung bei hohem pH intakt hielt. Die citraconylierten Peptide sind löslich und wurden durch Chromatographie in reverser Phase in basischer Lösung gereinigt, wobei reverse-Phase-Säulenpackungen auf Polymerbasis eingesetzt wurden.

Restriktionskartierung des D-HicDH-Gens auf dem L. casei-Genom

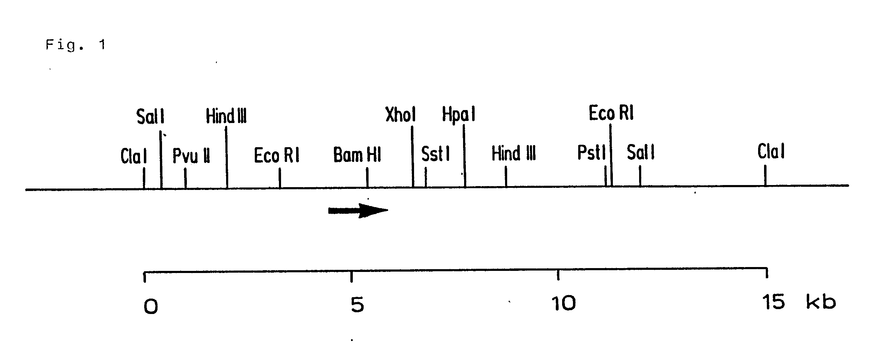

Die optimalen Hybridisierungsbedingungen für die 65-mer Oligonukleotid-Sonde wurden durch verschiedene genomische Southern-Blot Experimente gefunden. Wenn die Filter bei 50°C in 6 x SSC hybridisiert und gewaschen wurden, konnte man jeweils nur eine Bande für jede der Restriktionsendonukleasen-Verdauungen sehen. Einfache und doppelte Verdauungen von chromosomaler DNA durch Restriktionsendonukleasen wurden auf Agarosegelen aufgetrennt, und die Größe der hybridisierenden Fragmente wurde durch Soutern-Blotting bestimmt. Aus diesen Daten wurde eine Restriktions-Karte der Region auf dem L. Casei-Genom erstellt, die das D-HicDH-Gen enthält (Fig. 1).

## Klonieren des D-HicDH-Gens

1 mg chromosomale DNA von L. casei wurde mit ClaI verdaut und auf 0,8 %igem präparativem Agarosegel aufgetrennt. DNA-Fragmente mit einer Größe von etwa 15 kb wurden aus dem Gel herausgeschnitten, und die DNA wurde durch Elektroelution isoliert. Diese Fraktion wurde mit XhoI verdaut, und man isolierte Fragmente mit einer Größe von 6,5 kb. Das Eluat enthielt nur fünf verschiedene ClaI/XhoI-Fragmente, was sich nach Verdauung einer Probe dieser DNA-Fragmente durch BamHI zeigen ließ (die Daten sind hier nicht gezeigt). Die gereinig ten DNA-Fragmente wurden an durch ClaI, XhoI verdautem pBR322 mittels ligiert und in E. coli DH1 transformiert. Die Zellen wurden auf SR-Platten mit 50 µg/ml Ap ausplattiert. Die Plasmid-DNA wurde aus 40 der gebildeten Klone isoliert und mit ClaI und BamHI verdaut. Vier dieser Klone enthielten eine Insertion im Vektor, und einer dieser vier Klone zeigte nach Verdauung mit ClaI und BamHI die erwarteten Fragmentgrößen. In einem Southern-Blot Experment hybridisierte dieser Klon stark mit der 65-mer-Oligonucleotid-Sonde. Die Restriktions-Kartierung des insertierten DNA-Fragments mit BamHI, EcoRI, HindIII, PvuII und SalI und anschließendes Southern-Blotting zeigte Übereinstimmung der Lokalisation der Restriktionsstellen mit der chromosomalen Karte. Dieser Klon (pHL 1), der das 6,5 kb Fragment der chromosomalen DNA von L. Casei in pBR322 trägt, ließ sich infolge seines abnormalen Wachstumsverhaltens nur schwer kultivieren. Wenn man eine über Nacht inkubierte Kultur 1:100 in frischem Medium verdünnte, konnte man während der nächsten 10 Stunden kein Wachstum beobachten, während eine ähnlich behandelte Kultur von E. coli DH1 (pBR322) während dieser Zeit bis zur spätlogarithmischen Phase wuchs. Nach 24 Stunden besaßen beide Kulturen dieselbe optische Dichte ($OD_{550}$ = 28 bei Züchtung in SR Medium). Dieses Experiment wurde fünfmal wiederholt, indem beide Kulturen täglich verdünnt wurden, und die Ergebnisse waren jedesmal dieselben. Die Deletions-Klone, die für die Sequenzierung des D-HicDH-Gens erzeugt wurden, zeigten diese 10 h log-Phase nicht, obwohl sie D-HicDH in einer Menge von 3-5 % des gesamten Zellproteins produzierten.

## Niedrige Klonierungsfrequenz

Die primäre Isolierung eines D-HicDH-Klons kann als ineffizienter Vorgang gewertet werden, der aber nicht durch eine nachteilige Wirkung des Genprodukts selbst verursacht ist. Die DNA-Region, die lange Unterbrechungen im Wachstum frisch inokulierter Kulturen verursacht, ist mit dem D-HicDH-Gen im 3,3 kb ClaI, EcoRI-Fragment verknüpft. Zum derzeitigen Zeit punkt läßt sich über die Ursache dieser Schwierigkeit im Lichte ähnlicher Probleme beim Klonieren von Lactobacillus-oder gram-positiven Genen, die in der Literatur erwähnt sind, nur spekulieren. Copeland et al. (1987) berichtet, daß die anfangs auftretende Schwierigkeit beim Klonieren von Histidin-Decarboxylase aus Lactobacillus 30a in E. coli in Zusammenhang mit der Präsenz eines sehr starken Promotors stand (15 mal so stark wie der vollständig induzierte lac-Promotor), der zu der homologen Gruppe von grampositiven Promotoren gehörte, die auch in E. coli exprimiert werden (Graves und Rabinovitz, 1986). Diese Homologie-Sequenz ist eine verlängerte Sequenz im Vergleich zum E. coli-Standard -Equivalent insofern, als es hochkonservierte Oligo-(A)-Regionen bei -45 und -3 bis -7 gibt. Die TTGACA-Sequenz bei -35 und die TG-TATAAT-Sequenz (-10) sind ebenfalls stärker konserviert. Diese sind Merkmale der extrem starken T5-Bakteriophagen-Promotoren, die nur in speziellen Vektoren mit sehr effizienten Terminations-Signalen kloniert werden konnten (Stauber und Bujard, 1982; Gentz und Bujard, 1985). In Hinblick auf dieses große Problem ist es vielleicht signifikant, daß nur über sechs andere funktionelle Lactobacillus-Gen-Klonierungen berichtet wurde (Davies und Gronenborn, 1982, Vanderslice et al., 1986, Lee et al., 1982, Chassy et al., 1982, Williams et al., 1984, Alpert und Chassy, 1988).

## Gensequenz

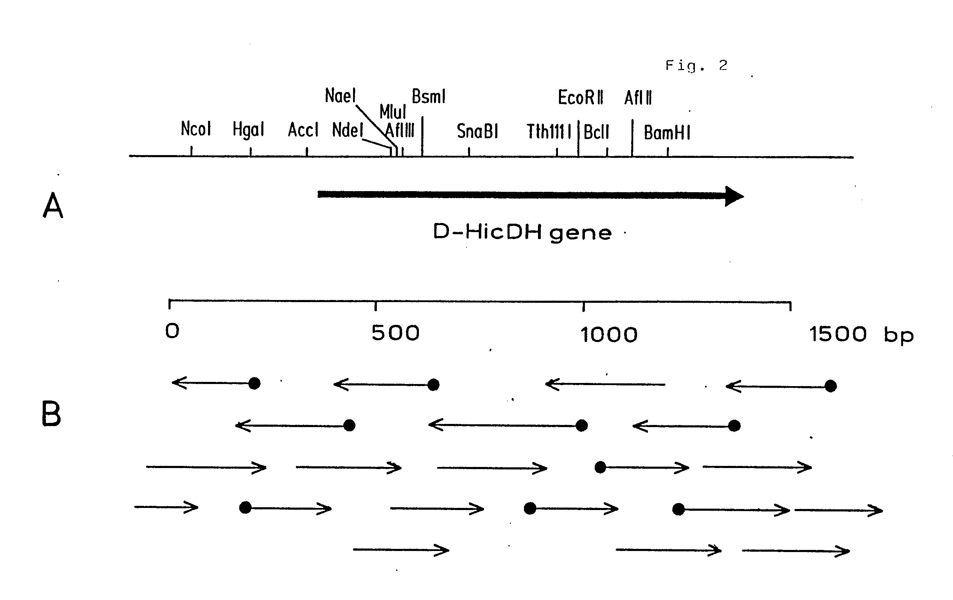

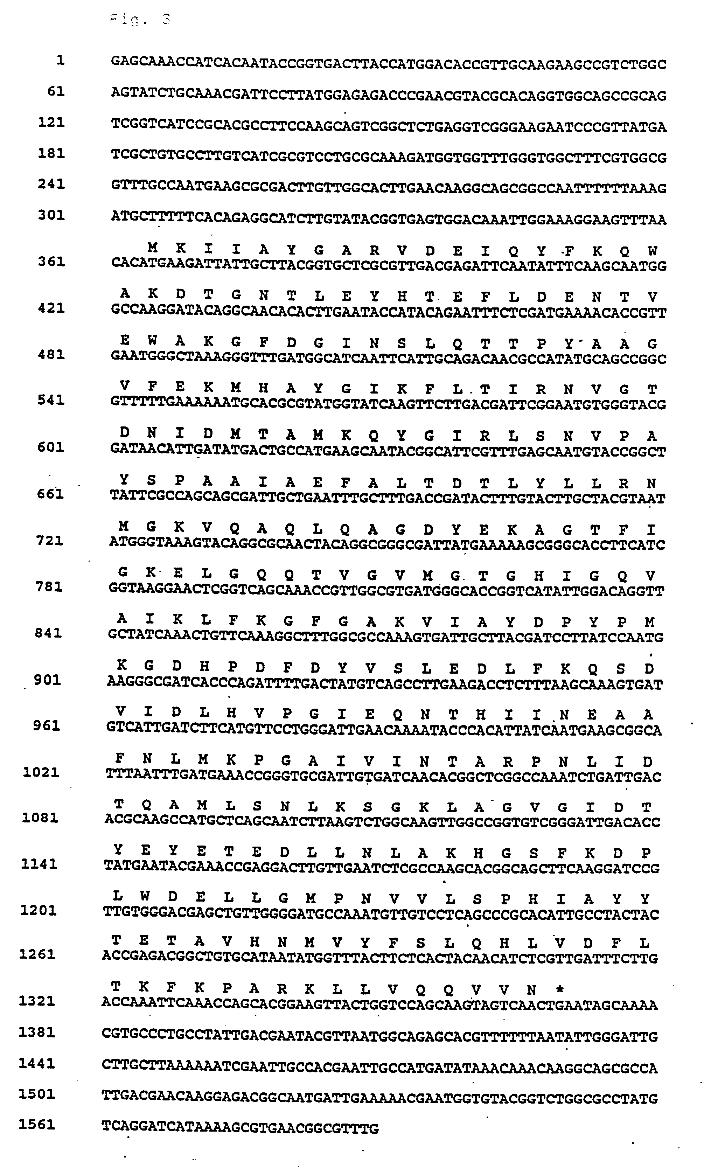

Die Sequenzierungs-Strategie und die vollständige Nukleotid-Sequenz des D-HicDH-Gens sind in Fig. 2 und 3 dargestellt. Die Codon-Verwendung beim D-HicDH-Gen wurde mit Daten aus anderen Lactobacillus-Genen verglichen (Tab. 2). Bisher wurden die Codons AGA/G (Arg) und UAG (ter) nicht entdeckt. Es sollte angemerkt werden, daß AGA/G in Wirbeltier-Mitochondrien als Terminations-Codon verwendet werden (Review: Fox, 1987). Obwohl keine Prokaryonten-Gruppe beschrieben wurde, die diesen Variationstypus bei der Codon-Benutzung besitzt, muß man dies weiterhin als offene Frage betrachten, bis man Daten (d.h. Fremdgen-Expression) darüber gewonnen hat, wie oder ob diese Codons in Lactobacillus verwendet werden (benutzbar sind).

Die mutmaßliche Shine-Dalgarno-Sequenz ist in Tabelle 2 mit bekannten Sequenzen für andere Lactobacillus-Gene verglichen worden. Die D-HicDH-Sequenzen stimmen in der Homologie-Sequenz überein, von der man annehmen sollte, daß sie sowohl in gram-positiven als auch in gram-negativen Bakterien erkannt wird.

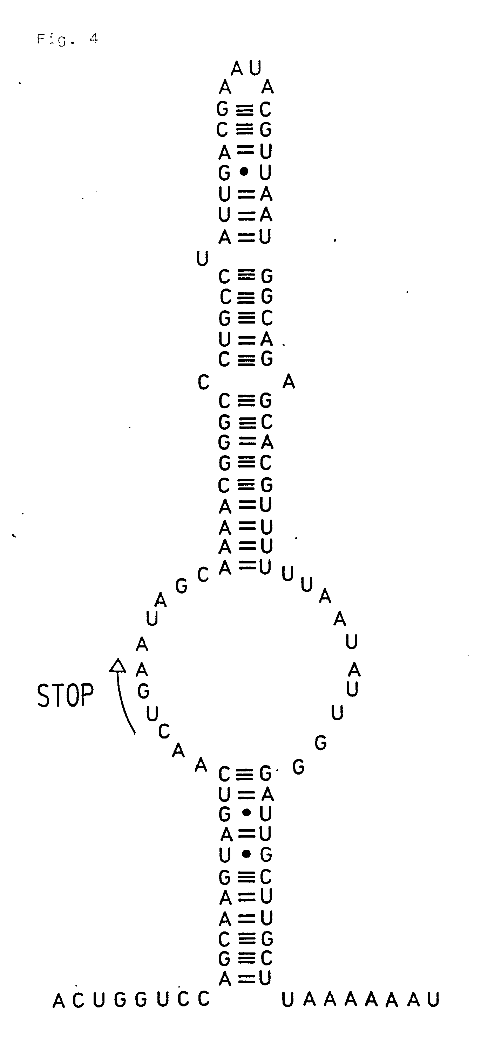

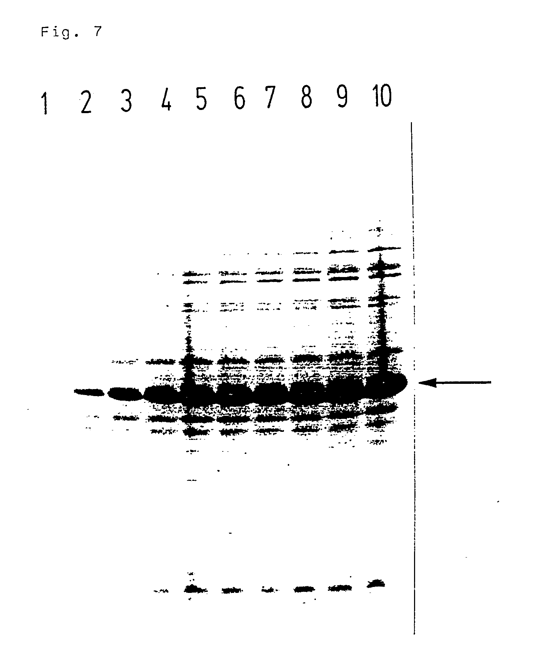

Unterhalb (strangabwärts) der codierenden Region befindet sich eine Anzahl von invertierten Wiederholungen. Eine mögliche große Schlaufenstruktur ist in Fig. 4 dargestellt. Die Schlaufensturktur repräsentiert einen typischen rho-unabhängigen Terminator (Typ I) (Review: Friedman et al., 1987). Angesichts der großen Mengen an D-HicDH, die in der Zelle mit dem pJLA 601-Vektor (Fig. 7) induzierbar sind (Fig. 7), scheint die Effizienz der Shine-Dalgarno-Sequenz und die Stabilität der messenger-RNA, die man erwarten sollte und die zumindest teilweise durch eine stabile Sekundärstruktur am 3'-Ende der m-RNA ((Newbury et al., 1987) bestimmt werden sollte, hoch zu sein.

## Evolutionäre Verwandtschaft von D-HicDH zu anderen Dehydrogenasen.

Die Aminosäuresequenz der D-HicDH wurde mit den Sequenzen aller Denhydrogenasen in der EMBL-Datenbank (Version 12) verglichen. Es fand sich keine signifikante Homologie (die 15% Homologie über den Gesamtbereich hinweg überschritt) zu irgendeiner anderen Dehydrogenase. Beim Suchen nach kürzeren Homologie-Regionen zeigten viele Dehydrogenasen Homologien zu den Aminosäureresten 146-180 der D-HicDH. Die entsprechenden Regionen der anderen Dehydrogenasen sind Teil der NADH-Bindungsstelle. Dieser strukturell stark konservierte Bereich besteht aus sechs ß-Faltblattstrukturen, die durch α-Helices verknüpft sind (Reviews siehe Rossmann et al., 1974, 1975). Die ßA-, αB- und ßB-Strukturelemente der D-HicDH lassen sich wegen der Ähnlichkeit in der Aminosäuresequenz zuordnen (Tabelle 4). In den Anderen Teilen der NADH-Bindungsregion (ßC, ßD, αD, αE, ßE und ßF) sind die Strukturen konserviert, obwohl die Aminsäure-Sequenz mehr Divergenz zeigt (nach Chou und Fasman (1974), nicht gezeigt). Eine Zuordnung dieser entsprechenden Regionen der D-HicDH wurde deshalb als äußerst spekulativ betrachtet.

## Expression des D-HicDH-Gens in E. coli

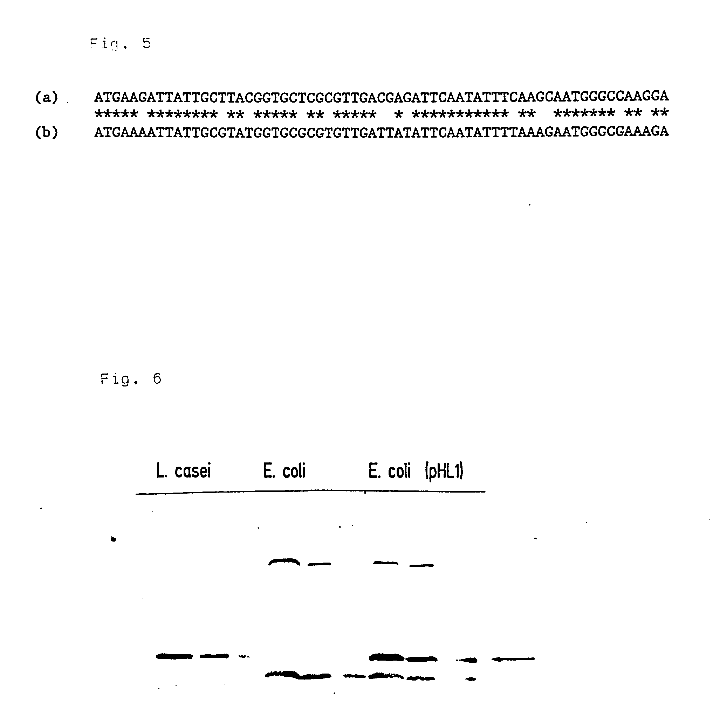

Der Western-Blot (Fig. 6) zeigt, daß das D-HicDH-Gen in E. coli exprimiert wird, wahrscheinlich von seinem natürlichen Lactobacillus-Promotor. Das in E. coli produzierte Enzym besitzt offensichtlich dasselbe Molekulargewicht wie das in L. casei produzierte. Es wurden keine Abbauprodukte beobachtet. Die Aktivität der D-HicDH wurde im löslichen Zellprotein gemessen. Die spezifische Aktivität im L. casei-Extrakt betrug 0,15 U/mg, und in E. coli DH1 als Kontrolle konnte keine D-HicDH-Aktivität gemessen werden. Das Klonieren des D-HicDH-Gens im Expressionsvektor pJLA 601 führt zu einem Klon, der D-HicDH in einer Menge von über 60 % des gesamten Zellproteins produziert (Fig. 7). D-HicDH akkumuliert in Form großer Einschlußkörper in den Zellen, und die Enzymaktivität (3,5 U/mg) im löslichen Zellprotein beträgt etwa 5% des erwarteten Wertes.

## SCHLUßFOLGERUNGEN

Es gibt zwei Ansätze, um mittels Oligonukleotid-Sonden nach Genen zu suchen, wie von Woods (1984) und Wood (1987) diskutiert. Die eine setzt eine Mischung von kurzen Oligonukleotiden (Größe 11-20 Basen) ein, um alle möglichen Codon-Varianten jeder Aminosäure zur Passung zu bringen. Die andere beruht auf langen Einzelsequenz-Sonden (Größe 30-40 Basen). Mit dem letztgenannten Verfahren, das für das Sondieren nach dem D-HicDHGen verwendet wurde, werden die wahrscheinlichsten Nukleotide für jedes Codon auf der Grundlage der Sequenz für das Staphylokokken-Lipase-Gen ausgewählt (Götz et al., 1985).

Der Vorteil dieses Verfahrens ist, daß ein oder zwei Fehler in der Aminsäure-Sequenz toleriert werden können (Pennica et al., 1984; Ullrich et al., 1985).

Die 65-mer-Oligonukleotid-Sonde stimmt mit der Genom-Sequenz des D-HicDH-Gens zu 80 % überein (52 von 65), wobei die längste homologe Region 11 Basen lang ist (Fig. 5). Die Differenzen an Position 34 und 36 wurden durch einen Artefakt verursacht, der während der N-terminalen Protein-Sequenzierung auftrat. Die Genom-Sequenz enthält Glu$_{12}$, während beim Sequenzieren des Proteins die Position mit Tyr gekennzeichnet worden war. Da das Protein vor der N-terminalen Sequenzierung mit Methanol/HCl versetzt worden war, könnte sich bei dieser Behandlung der Methylester von Glu gebildet haben, der eine falsche Interpretation der Daten verursacht. Ansonsten wurden keine Diskrepanzen zwischen den Peptid-Sequenzen und der Genom-Sequenz des D-HicDH-Gens gefunden.

Abkürzungen:

ADH = Alkohol-Dehydrogenase, Ap = Ampicillin, ATP = Adenosin-5'-triphosphat, C-terminal = carboxy-terminal, dATP = Desoxyadenosin-5'-triphosphat, D-HicDH = D-2-Hydroxyisocapronsäure-Dehydrogenase, DSM = Deutsche Sammlung von Mikroorganismen und Zellkulturen, EDTA = Ethylendiamintetraessigsäure, Exo III = Exonuklease III des Lambda-Bakteriophagen, FPLC = Hochdruckflüssigkeitschromatographie unter schnellem Fluß, GADPH = Glyceraldehyd-3-phosphat-Dehydrogenase, HEPES = N-2-Hydroxyethylpiperazin-N-2-ethansulfonsäure, kb = Kilobasenpaare, kDA = Kilodalton, lac = Lactose-Operon von Escherichia coli, LDH = D-Lactat-Dehydrogenase, LeuDH = L-Leucin-Dehydrogenase, L-HicDH = L-2-Hydroxyisocapronsäure-Dehydrogenase, N-terminal = amino-terminal, PAGE = Polyacrylamid-Gelelektrophorese, $P_L P_R$ = "linke" und "rechte" Promotoren der Kombatibilitätsregion von Lambda, SDS = Natriumdodecylsulfat, T4 = T4-Bakteriophage, λ = Lambda-Bakteriophage.

Tabelle 1:

Aminosäure-Teilsequenzen, bestimmt durch Protein-Sequenzierung nach proteolytischer Verdauung von D-HicDH. Homogene D-HicDH, hergestellt nach Kalwass et al. (1987), wurde nach folgenden Verfahren verdaut: Die Cyanogenbromid-Spaltung wurde 10 Stunden lang bei Raumtemperatur mit einem 1000 fachen molaren Überschuß bezüglich des Gesamtmethionins in 70 %iger Ameisensäure im Anschluß an eine 72-stündige Reduktion in 0,1 M 2-Mercaptoethanol bei Raumtemperatur und Vakuum-Trocknen durchgeführt. Die Lysyl-Reste wurden durch nach Cruickshank et al. (1974) durchgeführte Citraconylierung geschützt, mit anschließender Dialyse gegen 0,1 M Ammoniumhydrogencarbonat. Die tryptische Verdauung nativer oder citraconylierter D-HicDH wurde mit Hilfe von 1 Gewichts-% TPCK-Trypsin (Sigma) bei 37°C in 0,1 M Ammoniumhydrogencarbonat drei Stunden lang und nach neuerlicher Zugabe von 1 % Trypsin drei weitere Stunden lang durchgeführt. Die Endoproteinase Arg-C (Boehringer) ließ man 24 Stunden lang in einer Menge von 5 Gew.-%, bezogen auf die D-HicDH, in 0,1 M Ammoniumhydrogencarbonat bei 37°C einwirken. Nach der proteolytischen Verdauung wurden die Proben lyophilisiert und einer Chromatographie auf reverser Phase auf einer Nucleosil $C_{18}$ 10-μm-Säule (Macherey und Nagel) oder einer Hypersil $C_4$ 5-μm-Säule (Shandon) unterworfen, wobei eine Gradienten-Elution mit Acetonitril oder Isopropanol in 0,1 %iger (v/v) Trifluoressigsäure verwendet wurde. Die tryptischen Verdauungen der citraconylierten D-HicDH wurden direkt für die Chromatographie auf reverser Phase auf einer Poly-F-Säule (DuPont) mit Acetonitril-Gradienten-Elution in 1 %igem (v/v) Triethylamin eingesetzt. Die Peptide wurden in einem Protein-Sequenz-Analysator Modell 470 A (Applied Biosystems) unter Bindung an Polybren sequenziert.

Tabelle 2:

Codon-Benutzung in Lactobacillus. A = Lactobacillus casei, Dihydrofolat-Reduktase-Gen (Andrews et al., 1985), B = Lactobacillus 30 a, Histidin-Decarboxylase-Gen (Vanderslice et al., 1986), C = ORF 1 aus dem Insertionselement ISL 1, isoliert aus Lactobacillus casei-Bacteriophagen (Shimizu-Kadota et al., 1985), D = ß-D-Phosphogalactosid-Galactohydrolase-Gen von L. casei (Porter und Chassy, 1988), E = Faktor III-lac Gen von L. casei (Alpert und Chassy, 1988), F = Lactobacillus casei ssp. pseudoplantarum, D-2-Hydroxyisocapronsäure-Dehydrogenase-Gen (siehe diese Erfindung).

Tabelle 3:

Shine-Dalgarno-Sequenzen für Lactobacillus-Gene. Die Bezeichnungen entsprechen denen aus Tabelle 2.

Tabelle 4:

Zuordnung von NADH-Bindungsstruktur-Elementen verschiedener Dehydrogenasen nach Rossmann (1975). * = D-HicDH-Sequenz stimmt mit der Homologie-Region ("Consensus-sequence") überein. + = konservativer Aminosäure-Austausch in der D-HicDH-Sequenz bezüglich der Homologie-Region ("Consensus-sequence"). Die Zuordnung basiert auf Struktur-Daten aus Röntgen-Kristallographie (Ohlsson et al., 1974, Rossmann et al., 1974), für die Sequenzen der GAPDH aus Schwein und Hefe, der LDH aus L. casei, der ADH aus D. melanogaster und der ADH aus Ratte. Die D-HicDH-Sequenz aus L. casei wird mittels Sequenz-Vergleich zugeordnet. Für die D-HicDH stimmt die Zuordnung mit der Voraussage für die Sekundärstruktur überein (Garnier et al., 1978, Chou und Fasman, 1974).
a) Harris und Perham (1968), b) Davidson et al. (1967), c) Holland und Holland (1979), d) Taylor (1977), e) Hensel et al. (1983), f) Benyajati et al. (1981), g) Jörnvall (1970), h) Bränden et al (1975).
Fig. 1 Restriktionskarte des L. casei-Genoms im Bereich des D-HicDH-Gens. Der Pfeil markiert das D-HicDH-Gen.
Fig. 2
A) Restriktionskarte des D-HicDH-Gens. Es sind nur einmal schneidende Enzyme gezeigt.
B) Sequenzierungs-Strategie. Ein EcoRI, PvuII-Fragment aus pHL1 wurde in mit EcoRI, PvuII gespaltenen pGEM3 kloniert. Nach Spaltung mit EcoRI und PstI, Verdauung mit Exonuklease III und Mungobohnen-Nuklease, Ligieren und Transformieren in E. coli DH1 wurde ein Satz überlappender Klone generiert, wie unter "Methoden" beschrieben. Ein Stück des komplementären DNA-Strangs wurde durch Klonieren eines EcoRI, BamHI-Fragments aus pHL1 in mit EcoRI, BamHI gespaltenen pGEM4 und Sequenzieren vom SP6-Primer sequenziert. Die verbliebenen Lücken wurden unter Verwendung kurzer (etwa 20 Basen langer) synthetischer Oligonukleotid-Primer sequenziert, die auf den bekannten Sequenzen basierten. Sequenzierungsreaktionen, die mit synthetischen Primern begannen, sind durch einen Punkt auf dem Pfeil gekennzeichnet.
Fig. 3 Vollständige Nukleotid- und entsprechende Aminosäure-Sequenz des D-HicDH-Gens.
Fig. 4 Mögliche Sekundärstruktur am 3'-Ende des D-HicDH-Gens. Die Sequenz 1376 bis 1425 in Fig. 3, d.h. der obere Teil der hier gezeigten Sekundärstruktur, ist einem rho-unabhängigen Terminator ähnlich, wie er in E. coli gefunden wurde (Friedman et al., 1987).
Fig. 5 Zuordnung der Genom-Sequenz des D-HicDH-Gens (a) zur Sequenz der 65-mer-Oligonukleotid-Sonde (b).
Fig. 6 Western-Blot. Das gesamte Zellprotein von L. casei, E. coli DH1 und E. coli DH1 (pHL1) wurde auf einen 12,5 % Polyacrylamid-Gel aufgetrennt und ein Nitrocellulose-Filter geblottet. Von jeder Spezies wurden drei verschiedene Proteinkonzentrationen, jeweils um den Faktor zwei erniedrigt, (von links nach rechts) auf dem Gel aufgetragen. Der Pfeil weist auf die D-HicDH-Bande.
Fig. 7 Expression des D-HicDH-Gens unter Kontrolle von $\lambda P_R$-und $P_L$-Promotoren in E. coli DH1. Das D-HicDH-Gen wurde in die SphI-Stelle des Polylinkers des Expressionsvektors pJLA 601 kloniert. Der gebildete Klon wurde in SR-Medium bei 30°C bis zu einer $OD_{550} = 0{,}4$ gezüchtet und durch eine Temperaturerhöhung auf 42°C induziert. Das gesamte Zellprotein aus Teilen der Kultur wurden auf einem 12,5 % Polyacrylamid-Gel aufgetrennt. Spur 1: vor der Induktion; Spur 2: 30 Minuten nach der Induktion; die Zeitdifferenz zwischen den folgenden Spuren beträgt 30 min. Der Pfeil weist auf die D-HicDH-Bande.

Tabelle 1

| Spaltungs-Methode | durch automatisierten Edman-Abbau identifizierte Reste |
|---|---|
| Cyanogenbromid | 65 - 74; 88 - 117; 121 - 138; 152 - 177; 180 - 184 224 - 237; 288 - 298; 308 - 323 |
| Trypsin + native D-HicDH | 1 - 9; 181 - 234 |
| Trypsin + citraconylierte D-HicDH | 10 - 30; 119 - 145; 237 - 257 |
| Endoproteinase Arg-C | 94 - 121 |

Tabelle 2

| AA | Codon | A | B | C | D | E | F | Total |
|----|-------|---|---|---|---|---|---|-------|
| A (Ala) | GCA | 3 | 9 | 1 | 5 | 5 | 4 | 27 |
| | GCU | 6 | 17 | 2 | 9 | 7 | 10 | 51 |
| | GCC | 1 | 0 | 8 | 17 | 3 | 8 | 37 |
| | GCG | 5 | 1 | 1 | 10 | 1 | 7 | 25 |
| R (Arg) | AGA* | 0 | 0 | 0 | 0 | 0 | 0 | 0 |
| | AGG* | 0 | 0 | 0 | 0 | 0 | 0 | 0 |
| | CGA | 1 | 0 | 0 | 4 | 1 | 0 | 6 |
| | CGU | 2 | 10 | 4 | 5 | 0 | 2 | 23 |
| | CGC | 3 | 0 | 2 | 3 | 2 | 1 | 11 |
| | CGG | 2 | 1 | 1 | 5 | 1 | 3 | 13 |
| N (Asn) | AAU | 2 | 4 | 3 | 13 | 1 | 12 | 35 |
| | AAC | 1 | 8 | 2 | 1 | 3 | 5 | 20 |
| D (Asp) | GAU | 14 | 15 | 3 | 25 | 8 | 14 | 79 |
| | GAC | 1 | 10 | 1 | 12 | 0 | 7 | 31 |
| C (Cys) | UGU | 0 | 1 | 0 | 1 | 0 | 0 | 2 |
| | UGC | 0 | 1 | 0 | 1 | 0 | 0 | 2 |
| Q (Gln) | CAA | 3 | 9 | 2 | 18 | 10 | 24 | 46 |
| | CAG | 6 | 0 | 1 | 6 | 4 | 6 | 23 |
| E (Glu) | GAA | 5 | 22 | 6 | 23 | 9 | 13 | 78 |
| | GAG | 2 | 0 | 2 | 10 | 2 | 4 | 20 |
| G (Gly) | GGA | 0 | 0 | 1 | 4 | 0 | 1 | 6 |
| | GGU | 5 | 25 | 1 | 10 | 3 | 9 | 53 |
| | GGC | 5 | 3 | 0 | 13 | 3 | 13 | 37 |
| | GGG | 0 | 0 | 0 | 6 | 0 | 4 | 10 |
| H (His) | CAU | 6 | 1 | 1 | 15 | 2 | 5 | 30 |
| | CAC | 1 | 1 | 0 | 6 | 1 | 5 | 14 |
| I (Ile) | AUU | 3 | 15 | 1 | 16 | 3 | 16 | 54 |
| | AUC | 2 | 5 | 1 | 7 | 3 | 6 | 24 |
| | AUA | 0 | 0 | 0 | 1 | 0 | 0 | 1 |
| L (Leu) | UUA | 7 | 7 | 4 | 3 | 0 | 1 | 22 |
| | UUG | 3 | 7 | 2 | 13 | 3 | 13 | 41 |
| | CUA | 0 | 0 | 1 | 3 | 1 | 3 | 8 |
| | CUU | 0 | 4 | 1 | 3 | 4 | 4 | 16 |
| | CUC | 0 | 1 | 1 | 3 | 1 | 7 | 13 |
| | CUG | 3 | 0 | 1 | 7 | 0 | 4 | 15 |
| K (Lys) | AAA | 5 | 3 | 6 | 13 | 2 | 10 | 39 |
| | AAG | 4 | 17 | 4 | 12 | 4 | 13 | 54 |
| M (Met) | AUG | 3 | 12 | 2 | 11 | 9 | 11 | 48 |
| | CCC | 1 | 0 | 0 | 3 | 0 | 0 | 4 |
| | CCG | 2 | 0 | 1 | 9 | 0 | 4 | 16 |
| S (Ser) | UCA | 0 | 9 | 1 | 5 | 1 | 2 | 18 |
| | UCU | 0 | 5 | 2 | 4 | 0 | 1 | 12 |
| | UCC | 1 | 2 | 2 | 2 | 0 | 0 | 7 |
| | UCG | 0 | 0 | 0 | 4 | 0 | 1 | 5 |
| | AGU | 2 | 4 | 3 | 6 | 1 | 1 | 17 |
| | AGC | 1 | 2 | 0 | 3 | 0 | 5 | 11 |
| T (Thr) | ACA | 2 | 3 | 1 | 8 | 2 | 4 | 20 |
| | ACU | 1 | 12 | 0 | 2 | 1 | 2 | 18 |
| | ACC | 7 | 1 | 2 | 10 | 3 | 10 | 33 |
| | ACG | 4 | 0 | 0 | 5 | 2 | 6 | 17 |
| W (Trp) | UGG | 4 | 6 | 1 | 10 | 0 | 3 | 24 |
| Y (Tyr) | UAU | 5 | 7 | 3 | 19 | 1 | 8 | 43 |
| | UAC | 0 | 8 | 2 | 6 | 1 | 9 | 26 |
| V (Val) | GUA | 1 | 2 | 1 | 2 | 0 | 3 | 9 |
| | GUU | 8 | 14 | 2 | 11 | 3 | 9 | 47 |
| | GUC | 5 | 0 | 0 | 4 | 1 | 6 | 16 |
| | GUG | 2 | 1 | 0 | 12 | 2 | 5 | 22 |
| STOP | UGA | 0 | 0 | 0 | 0 | 0 | 1§ | 1 |
| | UAG* | 0 | 0 | 0 | 0 | 0 | 0 | 0 |
| | UAA | 1 | 1 | 1 | 1 | 1 | 0 | 5 |
| Total | | 164 | 312 | 94 | 475 | 113 | 336 | 1 494 |

* bisher in Lactobacillus nicht benutzt

§ unrepresentative Häufigkeit im D-HicDH-Gen

Tabelle 3

| Zitat | Sequenz |
|-------|---------|
| F | T G G A A A G G A A G T T T A A C A - C - A T G . . |
| B | T T A T T A G G A G G T C T A A T T - - - A T G . . |
| D | T T A A C A G G A G G T T A T T A A G C A A T G . . |
| E | T G A A T C G G A G G G A A A A T G - - - A T G . . |
| A | C T C A A A G G A G G G G T C T C A - - - A T G . . |
| C | C C G A A A G G A G T C C T C A A A - - - A T G . . |
| Überein-<br>stimmung | T t a A a A G G A G G t N T a A N A - - - A T G . . |

EP 0 351 575 A2

## Tabelle 4

. Sekundärstrukturelement

| Dehydrogenase | Ursprung | βA, | αB | βB | Zitat |
|---|---|---|---|---|---|
| GAPDH | Schwein (Muskel) | VKVGVD<u>G</u>F<u>G</u>RI<u>G</u>RLVTRAAFNSGKV<u>D</u>IVAIN<u>D</u>PFIDL | | | a) |
| GAPDH | Hummer (Muskel) | SKIGID<u>G</u>F<u>G</u>RI<u>G</u>RLVLRAALSCG-A<u>Q</u>VVAVN<u>D</u>PFIAL | | | b) |
| GAPDH | Hefe | VRVAIN<u>G</u>F<u>G</u>RI<u>G</u>RLVMRIALSRPNV<u>E</u>VVALN<u>D</u>PFITN | | | c) |
| LDH | Haifisch (Muskel) | NKITVV<u>G</u>V<u>G</u>AV<u>G</u>MACAISILMKDLA<u>D</u>EVALV<u>D</u>VMEDK | | | d) |
| LDH | L. casei | QKVILV<u>G</u>D<u>G</u>AV<u>G</u>SSYAFAMVLQGIA<u>Q</u>EIGIV<u>D</u>IFKDK | | | e) |
| ADH | D. melanogaster | NVIFVA<u>G</u>L<u>G</u>GI<u>G</u>LDTSKELLKRD-I<u>K</u>NLVIL<u>D</u>RIENP | | | f) |
| ADH | Pferd (Leber) | STCAVF<u>G</u>L<u>G</u>GV<u>G</u>LSVIMGCKAAG-A<u>A</u>RIIGV<u>D</u>INKDK | | | g) |
| ADH | Ratte (Leber) | STCAVF<u>G</u>L<u>G</u>GV<u>G</u>LSVVIGCKTAG-A<u>A</u>KIIAV<u>D</u>INKDK | | | h) |
| D-HicDH | L. casei | QTVGVM<u>G</u>T<u>G</u>HI<u>G</u>QVAIKLFKGFG--A<u>K</u>VIAY<u>D</u>PYPMK | | | erfindungs- gemäß |

**** * * **++    +   *   *   *  **   **+   *

$S^K_T V^G_A V$  $G^L_F G^{RI}_{GV} G^{RL}_{LS} V$  RAA$^K_L$  G-AA  V$^I_A$I$^V_N$DPF$^I_K$DK

Alpert, C.-A. and Chassy, B. M.: Molecular cloning and nucleotide sequence of the factor III lac gene of Lactobacillus casei. Gene 62 (1988) 277-288.

Andrews, J., Clore, G.M., Davies, R.W., Gronenborn, A.M., Gronenborn, B., Kalderon, D., Papadopoulos, P.C., Schäfer, S., Sims, P.F.G. and R. Stancombe: Nucleotide sequence of the dihydrofolate reductase gene of methotrexate-resistant Lactobacillus casei. Gene 35 (1985) 217-222.

Bae, H.S., Baek, Y.J., Kim, Y.K., Yoo, M. and Pack, Y.: Rapid and simple method for isolating plasmid DNA from lactic acid bacteria. Kor. J. Appl. Microbiol. Bioeng. 13 (1985) 289-296.

Benyajati, C., Place, A.R., Powers, D.A. and Sofer, W.: Alcohol dehydrogenase gene of Drosophila melanogaster: Relationship of intervening sequences to functional domains in the protein. Proc. Natl. Acad. Sci. USA 78 (1981) 2717-2721

Bolivar, F., Rodriguez, R.L., Greene, P.J., Betlach, M.C., Heynecker, H.L., Boyer, H.W., Crosa, J.H. and Falkow, S.: Construction and characterisation of new cloning vehicles. II. A multipurpose cloning system. Gene 2 (1977) 95-113.

Birnboim, H.L. and Doly, J.: A rapid alkaline extraction procedure for screening recombinant plasmid DNA. Nucl. Acids Res. 7 (1979) 1513-1523

Bränden, C.-J., Jörnvall, H.-, Eklund, H. and Furugren, B.: Alcohol dehydrogenases. In: Boyer, P.D. (ed.) The Enzymes 11 (1975) 104-190, Academic Press, New York, 1975

Chassy, B.M., Lee, L.-J., Hansen, J.B. and Jagusztyn-Krynicka, E.K.: Molecular Cloning of Lactobacillus casei lactose metabolic genes. Dev. Ind. Microbiol. 24 (1982) 97-107.

Chen, E.Y. and Seeburg, P.H.: Supercoil sequencing: A fast and simple method for sequencing plasmid DNA. DNA 4 (1985) 165-170

Chou, P.Y. and Fasman, G.D.: Prediction of protein conformation. Biochemistry 13 (1974) 222-245.

Copeland, W.C., Vanderslice, P. and Robertus, J. D. : Expression and characterisation of Lactobacillus 30 a histidine decarboxylase in Escherichia coli. Protein Engineering 1 (1987) 419-423.

Cruickshank W. H., Malchy B. L. and Kaplan H.: Diagonal chromatography for the selective purification of tyrosyl peptides. Can. J. Biochem. 52 (1974) 1013-1017

deMan, J.C., Rogosa, M. and Sharpe, M.E.: A medium for the cultivation of lactobacilli. J. Appl. Bact. 23 (1960) 130-135.

Davies, R.W. and Gronenborn, A.M.: Molecular cloning of the gene for dihydrofolate reductase from Lactobacillus casei. Gene 17 (1982) 229-233.

Davidson, B.E., Sajgo, M., Noller, H.F. and Harris, J.I.: Aminoacid sequence of glyceraldehyde 3-phosphate dehydrogenase from Lobster muscle. Nature 216 (1967) 1181-1185

Dixon, H. B. F. and Perham, R. N.: Reversible blocking of amino groups with citraconic anhydride. Biochem. J. 109 (1968) 312.314

Fox, D.T.: Natural variation in the genetic code. Ann. Rev. Genet. 21 (1987) 67-91.

Frank, R., Meyerhans, A., Schwellnus, K. and Blöcker, H.: Simultaneous synthesis and biological applications of DNA fragments: an efficient and complete methodology. Meth. Enz. 154 (1988) 201-249

Friedman, D.I., Imperiale, M.J. and Adhya, S.L.: RNA 3' end formation in the control of gene expression. Ann. Rev. Genet. 21 (1987) 453-488.

Garnier, J., Osguthorpe, D.J. and Robson, B.: Analysis of the accuracy and implications of simple methods for predicting the secondary structure of globular proteins. J. Mol. Biol. 120 (1978) 97-120.

Gentz, R. and Bujard, H.: Promoters recognised by Escherichia coli RNA polymerase selected by function: highly efficient promoters from bacteriophage T5. J. Bacteriol. 164 (1985), 70-77.

Götz, F., Popp, F., Korn, E. and Schleifer, K.H.: Complete nucleotide sequence of the lipase gene from Staphylococcus hyicus cloned in Staphylococcus carnosus. Nucl. Acids Res. 13 (1985) 5895-5906.

Graves, M.C. and Rabinowitz, J.C.: In vivo and in vitro transcription of the Clostridium pasteurianum ferredoxin gene. J. Biol. Chem. 261 (1986) 11409-11415.

Hanahan, D.: Studies on transformation of Escherichia coli with plasmids. J. Mol. Biol. 166 (1983) 557-580

Harris, J.I. and Perham, R.N.: Glyceraldehyde 3-phosphate dehydrogenase from pig muscle. Nature 219 (1968) 1025-1028

Hensel, R., Mayr, U. and Yang, C.: The complete primary structure of the allosteric L-lactate dehydrogenase from Lactobacillus casei. Eur. J. Biochem. 134 (1983) 503-511.

Henikoff, S.: Unidirectional digestion with exonuclease III creates targeted breakpoints for DNA sequencing. Gene 28 (1984) 351-359.

Holland, J.P. and Holland, M.J.: The primary structure of a glyceraldehyde-3-phosphate dehydrogenase gene from Saccharomyces cerevisiae. J. Biol. Chem. 254 (1979) 9839-9845.

Houmard J. and Drapeau G. R.: Staphylococcal protease: A proteolytic enzyme specific for glutamoyl bonds. Proc. Nat. Acad. Sci. 69 USA (1972) 3506-3509

Hummel, W., Schütte, H. and Kula, M.-R.: New enzymes for the synthesis of chiral compounds. In: Enzyme Engineering 7; Ann. N.Y. Acad. Sci. 434 (1984) 194-205.

Hummel, W., Schütte, H. and Kula, M.-R.: D-2-hydroxyisocaproate dehydrogenase from Lactobacillus casei. A new enzyme suitable for stereospecific reduction of 2-ketocarboxylic acids. Appl. Microbiol. Biotechnol. 21 (1985) 7-15.

Jörnvall, H.: Horse liver alcohol dehydrogenase - on the primary structure of the isoenzymes. Eur. J. Biochem. 16 (1970) 41-49.

Kallwaß H., Tsai H. and Schütte H.: Crystallisation and molecular properties of D-2-hydroxyisocaproate dehydrogenase from Lactobacillus casei. FEMS Microbiol. Lett. 43 (1987) 263-267

Khandjian, E.W.: Optimized hybridization of DNA blotted and fixed to nitrocellulose and nylon membranes. Biotechnology 5 (1987) 165-167.

Laemmli, U.K.: Cleavage of structural proteins during the assembly of the head of bacteriophage T4. Nature 227 (1970) 680-685.

Lee, L.-J., Hansen, J.B., Jagusztyn-Krynicka, E.K. and Chassy, B.M.: Cloning and expression of the β-D-phosphogalactoside galactohydrolase gene of Lactobacillus casei in Escherichia coli K-12. J. Bacteriol. 152 (1982) 1138-1146.

Monsen, T.J., Holm, S.E. and Burman, L.G.: A general method for cell lysis and preparation of deoxyribonucleic acid from streptococci. FEMS Microbiol. Lett. 16 (1983) 19-24.

Newbury, S.F., Smith, N.H., Robinson, E.C., Hiles, I.D. and Higgins, C.F.: Stabilization of translationally active mRNA by procaryotic REP sequences. Cell 48 (1987) 297-310.

Ohlsson, I., Nordström, B. and Brändén, C.-I.: Structural and functional similarities within the coenzymes binding domains of dehydrogenases. J. Mol. Biol. 89 (1974) 339-354.

Pennica, D., Nedwin, G.E., Hayflick, J.S., Seeburg, P.H., Derynck, R., Palladino, M.A., Kohr, W.J., Aggarwal, B.B. and Goeddel, D.V.: Human tumour necrosis factor: precursor structure, expression and homology to lyphotoxin. Nature 312 (1984) 724-729.

Porter, E.V. and Chassy, B.M.: Nucleotide sequence of the β-D-phosphogalactoside galactohydrolase gene of lactobacillus casei: comparison to analogous pbg genes of other Gram-positive organisms. Gene 62 (1988) 263-276.

Rossmann, M.G., Liljas, A., Brändén, C.-J. and Banaszak, L.J.: Evolutionary and structural relationships among dehydrogenases. In: Boyer, P.D. (ed.) The Enzymes 11 (1975) 61-102, Academic Press, New York, 1975.

Rossmann, M.G., Moras, D. and Olsen, K.W.: Chemical and biological evolution of a nucleotide-binding protein. Nature 250 (1974) 194-199.

Sanger, F., Nicklen, S. and Coulson, A.R.: DNA sequencing with chain-terminating inhibitors. Proc. Natl. Acad. Sci. USA 74 (1977) 5463-5467.

Schauder, B., Blöcker, H., Frank, R. and McCarthy J.E.G.: Inducible expression vectors incorporating the Escherichia coli atpE translational initiation region. Gene 52 (1987) 279-283

Schütte, H., Hummel, W. and Kula, M.-R.: L-2-hydroxyisocaproate dehydrogenase - a new enzyme from Lactobacillus confusus for the stereospecific reduction of 2-ketocarboxylic acids. Appl. Microbiol. Biotechnol. 19 (1984) 167-176.

Schütte, H., Hummel, W., Tsai, H. and Kula, M.-R.: L-Leucine dehydrogenase from Bacillus cereus - production, large scale purification and protein characterization. Appl. Microbiol. Biotechnol. 22 (1985) 306-317.

Shimizu-Kadota, M., Kiwaki, M., Hirokawa, H. and Tsuchida, N.: ISL1: a new transposable element in Lactobacillus casei. Mol. Gen. Genet. 200 (1985) 193-198.

Southern, E.M.: Detection of specific sequences among DNA fragments separated by gel electrophoresis. J. Mol. Biol. 98 (1975) 503-517.

Stüber, D. and Bujard, H.: Transcription from efficient promoters can interfere with plasmid replication and diminish expression of plasmid specified genes. EMBO J. 1 (1982) 1399-1404.

Tabor, S. and Richardson, C.C.: DNA sequence analysis with a modified bacteriophage T7 DNA polymerase. Proc. Natl. Acad. Sci. USA 84 (1987) 4767-4771.

Taylor, S.S.: Amino acid sequence of Dogfish muscle lactate dehydrogenase. J. Biol. Chem. 252 (1977) 1799-1806.

Towbin, H., Staehelin, T. and Gordon, J.: Electrophoretic transfer of proteins from polyacrylamide gels to nitrocellulose sheets: procedure and some applications. Proc. Natl. Acad. Sci. USA 76 (1979) 4350-4354

Tsai, H., Lerch, H.-P., Kalwass, H., Schütte, H., Hoppe, J. and Collins, J.: The D- and L-2-hydroxyisocaproate dehydrogenases from lactobacilli are structurally and immunologically unrelated enzymes. In: Neijssel, O.M., van der Meer, R.R. and Luyben, K.Ch.A.M. (eds.), Proc. 4th Eur. Congr. Biotechn. 1987 Vol. 2, Elsevier Science Publ., Amsterdam, 1987, pp. 228-231.

Ullrich, A., Bell, J.R., Chen, E.Y., Herrera, R., Petruzzelli, L.M., Dull, T.J., Gray, A., Coussens, L., Liao, Y.-C., Tsubokawa, M., Mason, A., Seeburg, P.H., Grunfeld, C., Rosen, O.M. and Ramachandran, J.: Human insulin receptor and its relationship to the tyrosine kinase family of oncogenes. Nature 313 (1985) 756-761.

Vanderslice, P., Copeland, W.C. and Robertus, J.D.: Cloning and nucleotide sequence of wild type and a mutant histidine decarboxylase from Lactobacillus 30a. J. Biol. Chem. 261 (1986) 15186-15191.

Wandrey, C., Fiolitakis, E., Wichmann, U. and Kula, M.-R.: L-amino acids from a racemic mixture of α-hydroxy acids. Enzyme Engineering 7, Ann. N.Y. Acad. Sci. 434 (1984) 91-94.

Williams, S.A., Hodges, R.A., Strike, T.L., Snow, R. and Kunkee, R.E.: Cloning the gene for the malolactic fermentation of wine from Lactobacillus delbrueckii in Escherichia coli and yeasts. Appl. Env. Microbiol. 47 (1984) 288-293.

Wood, J.W.: Gene cloning based on long oligonucleotide probes. Meth. Enzymol. 152 (1987) 443-447.

Woods, D.: Oligonucleotide screening of cDNA libraries. Focus 6 (1984) 1-3.

Zassenhaus, H.P., Butow, R.A. and Hannon, Y.P.: Rapid electroelution of nucleic acids from agarose and acrylamide gels. Anal. Biochem. 125 (1982) 125-130.

**Ansprüche**

1. D-HicDH-Gen, nämlich Positionen 364 bis 1362 oder bis 1365 oder bis 1368 oder bis 1371 oder bis 1374 oder bis 1377 der folgenden DNA-Sequenz:

```
301   ATGCTTTTTCACAGAGGCATCTTGTATACGGTGAGTGGACAAATTGGAAAGGAAGTTTAA

      M  K  I  I  A  Y  G  A  R  V  D  E  I  Q  Y  F  K  Q  W
361   CACATGAAGATTATTGCTTACGGTGCTCGCGTTGACGAGATTCAATATTTCAAGCAATGG

      A  K  D  T  G  N  T  L  E  Y  H  T  E  F  L  D  E  N  T  V
421   GCCAAGGATACAGGCAACACACTTGAATACCATACAGAATTTCTCGATGAAAACACCGTT

      E  W  A  K  G  F  D  G  I  N  S  L  Q  T  T  P  Y  A  A  G
481   GAATGGGCTAAAGGGTTTGATGGCATCAATTCATTGCAGACAACGCCATATGCAGCCGGC

      V  F  E  K  M  H  A  Y  G  I  K  F  L  T  I  R  N  V  G  T
541   GTTTTTGAAAAAATGCACGCGTATGGTATCAAGTTCTTGACGATTCGGAATGTGGGTACG

      D  N  I  D  M  T  A  M  K  Q  Y  G  I  R  L  S  N  V  P  A
601   GATAACATTGATATGACTGCCATGAAGCAATACGGCATTCGTTTGAGCAATGTACCGGCT

      Y  S  P  A  A  I  A  E  F  A  L  T  D  T  L  Y  L  L  R  N
661   TATTCGCCAGCAGCGATTGCTGAATTTGCTTTGACCGATACTTTGTACTTGCTACGTAAT

      M  G  K  V  Q  A  Q  L  Q  A  G  D  Y  E  K  A  G  T  F  I
721   ATGGGTAAAGTACAGGCGCAACTACAGGCGGGCGATTATGAAAAAGCGGGCACCTTCATC

      G  K  E  L  G  Q  Q  T  V  G  V  M  G  T  G  H  I  G  Q  V
781   GGTAAGGAACTCGGTCAGCAAACCGTTGGCGTGATGGGCACCGGTCATATTGGACAGGTT

      A  I  K  L  F  K  G  F  G  A  K  V  I  A  Y  D  P  Y  P  M
841   GCTATCAAACTGTTCAAAGGCTTTGGCGCCAAAGTGATTGCTTACGATCCTTATCCAATG

      K  G  D  H  P  D  F  D  Y  V  S  L  E  D  L  F  K  Q  S  D
901   AAGGGCGATCACCCAGATTTTGACTATGTCAGCCTTGAAGACCTCTTTAAGCAAAGTGAT

      V  I  D  L  H  V  P  G  I  E  Q  N  T  H  I  I  N  E  A  A
961   GTCATTGATCTTCATGTTCCTGGGATTGAACAAAATACCCACATTATCAATGAAGCGGCA

      F  N  L  M  K  P  G  A  I  V  I  N  T  A  R  P  N  L  I  D
1021  TTTAATTTGATGAAACCGGGTGCGATTGTGATCAACACGGCTCGGCCAAATCTGATTGAC

      T  Q  A  M  L  S  N  L  K  S  G  K  L  A  G  V  G  I  D  T
1081  ACGCAAGCCATGCTCAGCAATCTTAAGTCTGGCAAGTTGGCCGGTGTCGGGATTGACACC

      Y  E  Y  E  T  E  D  L  L  N  L  A  K  H  G  S  F  K  D  P
1141  TATGAATACGAAACCGAGGACTTGTTGAATCTCGCCAAGCACGGCAGCTTCAAGGATCCG

      L  W  D  E  L  L  G  M  P  N  V  V  L  S  P  H  I  A  Y  Y
1201  TTGTGGGACGAGCTGTTGGGGATGCCAAATGTTGTCCTCAGCCCGCACATTGCCTACTAC

      T  E  T  A  V  H  N  M  V  Y  F  S  L  Q  H  L  V  D  F  L
1261  ACCGAGACGGCTGTGCATAATATGGTTTACTTCTCACTACAACATCTCGTTGATTTCTTG

      T  K  F  K  P  A  R  K  L  L  V  Q  Q  V  V  N  *
1321  ACCAAATTCAAACCAGCACGGAAGTTACTGGTCCAGCAAGTAGTCAACTGAATAGCAAAA
```

wobei das Gen an beliebiger Position um 1, 2 oder 3 Basentriplets verkürzt oder verlängert sein kann oder wobei ein Basentriplet des Gens durch ein anderes Basentriplet ersetzt sein kann oder wobei ein oder mehrere Basentriplets durch ein dieselbe Aminosäure kodierendes Basentriplet ersetzt sein können.

2. DNA-Teilsequenz, nämlich Positionen 343, 344, 345 oder 346 bis jeweils 363 der DNA-Sequenz gemäß Anspruch 1, wobei die DNA-Teilsequenz an beliebiger Position um 1, 2 oder 3 Basen verkürzt oder verlängert sein kann oder wobei 1, 2 oder 3 Basen der DNA-Teilsequenz durch jeweils eine andere Base ersetzt sein können.

3. DNA-Teilsequenz, nämlich 51 bis 93 Basen aus dem Bereich ab Position 1363 bis maximal Position 1455 der DNA-Sequenz gemäß Anspruch 1, wobei die DNA-Teilsequenz an beliebiger Position um 1, 2 oder 3 basen verkürzt oder verlängert sein kann oder wobei 1, 2 oder 3 Basen der DNA-Teilsequenz durch jeweils eine andere Base ersetzt sein können, wobei jedoch der Positionsbereich von 1376 bis 1427 stets eingeschlossen ist.

4. Verwendung einer DNA-Teilsequenz gemäß Anspruch 2 zur Translationsinitiation für die Herstellung von Genprodukten in gram-positiven oder gram-negativen Mikroorganismen.

5. Verwendung einer DNA-Teilsequenz gemäß Anspruch 3 als Transkriptions-Terminator für die Herstellung von Genprodukten in gram-positiven oder gram-negativen Mikroorganismen.

6. Verwendung nach Anspruch 5, dadurch gekennzeichnet, daß die DNA-Teilsequenz angehängt am 3'-Terminus des Gens des gewünschten Genprodukts verwendet wird, ggf. als einziger Transkriptions-Terminator.

7. Verwendung nach einem der Ansprüche 4, 5 oder 6 in E. coli.

8. Expressionsvektoren zur Expression von gewünschten Genprodukten in gram-positiven oder gram-negativen Mikroorganismen, wie E. coli, umfassend eine DNA-Teilsequenz gemäß Anspruch 2 und/oder Anspruch 3.

9. Expressionsvektoren zur Expression von D-HicDH in E. coli, umfassend das D-HicDH-Gen gemäß Anspruch 1.

10. E.-coli-Stamm für die Herstellung von D-HicDH mit Hilfe eines das D-HicDH-Gen gemäß Anspruch 1 umfassenden Expressionsvektors.

11. E.-coli-Stamm nach Anspruch 10, nämlich DSM 4535.

12. D-HicDH der folgenden Aminosäuresequenz:

```
           M  K  I  I  A  Y  G  A  R  V  D  E  I  Q  Y  F  K  Q  W
    361  CACATGAAGATTATTGCTTACGGTGCTCGCGTTGACGAGATTCAATATTTCAAGCAATGG

           A  K  D  T  G  N  T  L  E  Y  H  T  E  F  L  D  E  N  T  V
    421  GCCAAGGATACAGGCAACACACTTGAATACCATACAGAATTTCTCGATGAAAACACCGTT

           E  W  A  K  G  F  D  G  I  N  S  L  Q  T  T  P  Y  A  A  G
    481  GAATGGGCTAAAGGGTTTGATGGCATCAATTCATTGCAGACAACGCCATATGCAGCCGGC

           V  F  E  K  M  H  A  Y  G  I  K  F  L  T  I  R  N  V  G  T
    541  GTTTTTGAAAAAATGCACGCGTATGGTATCAAGTTCTTGACGATTCGGAATGTGGGTACG

           D  N  I  D  M  T  A  M  K  Q  Y  G  I  R  L  S  N  V  P  A
    601  GATAACATTGATATGACTGCCATGAAGCAATACGGCATTCGTTTGAGCAATGTACCGGCT

           Y  S  P  A  A  I  A  E  F  A  L  T  D  T  L  Y  L  L  R  N
    661  TATTCGCCAGCAGCGATTGCTGAATTTGCTTTGACCGATACTTTGTACTTGCTACGTAAT

           M  G  K  V  Q  A  Q  L  Q  A  G  D  Y  E  K  A  G  T  F  I
    721  ATGGGTAAAGTACAGGCGCAACTACAGGCGGGCGATTATGAAAAAGCGGGCACCTTCATC

           G  K  E  L  G  Q  Q  T  V  G  V  M  G  T  G  H  I  G  Q  V
    781  GGTAAGGAACTCGGTCAGCAAACCGTTGGCGTGATGGGCACCGGTCATATTGGACAGGTT

           A  I  K  L  F  K  G  F  G  A  K  V  I  A  Y  D  P  Y  P  M
    841  GCTATCAAACTGTTCAAAGGCTTTGGCGCCAAAGTGATTGCTTACGATCCTTATCCAATG

           K  G  D  H  P  D  F  D  Y  V  S  L  E  D  L  F  K  Q  S  D
    901  AAGGGCGATCACCCAGATTTTGACTATGTCAGCCTTGAAGACCTCTTTAAGCAAAGTGAT
```

```
        V   I   D   L   H   V   P   G   I   E   Q   N   T   H   I   I   N   E   A   A
961     GTCATTGATCTTCATGTTCCTGGGATTGAACAAAATACCCACATTATCAATGAAGCGGCA

        F   N   L   M   K   P   G   A   I   V   I   N   T   A   R   P   N   L   I   D
1021    TTTAATTTGATGAAACCGGGTGCGATTGTGATCAACACGGCTCGGCCAAATCTGATTGAC

        T   Q   A   M   L   S   N   L   K   S   G   K   L   A   G   V   G   I   D   T
1081    ACGCAAGCCATGCTCAGCAATCTTAAGTCTGGCAAGTTGGCCGGTGTCGGGATTGACACC

        Y   E   Y   E   T   E   D   L   L   N   L   A   K   H   G   S   F   K   D   P
1141    TATGAATACGAAACCGAGGACTTGTTGAATCTCGCCAAGCACGGCAGCTTCAAGGATCCG

        L   W   D   E   L   L   G   M   P   N   V   V   L   S   P   H   I   A   Y   Y
1201    TTGTGGGACGAGCTGTTGGGGATGCCAAATGTTGTCCTCAGCCCGCACATTGCCTACTAC

        T   E   T   A   V   H   N   M   V   Y   F   S   L   Q   H   L   V   D   F   L
1261    ACCGAGACGGCTGTGCATAATATGGTTTACTTCTCACTACAACATCTCGTTGATTTCTTG

        T   K   F   K   P   A   R   K   L   L   V   Q   Q   V   V   N   *
1321    ACCAAATTCAAACCAGCACGGAAGTTACTGGTCCAGCAAGTAGTCAACTGAATAGCAAAA
```

wobei die folgenden Buchstaben bedeuten:

A = Alanin
D = Asparaginsäure
E = Glutaminsäure
F = Phenylalanin
G = Glycin
H = Histidin
I = Isoleucin
K = Lysin
L = Leucin
M = Methionin
N = Asparagin
P = Prolin
Q = Glutamin
R = Arginin
S = Serin
T = Threonin
V = Valin
W = Tryptophan
Y = Tyrosin

wobei das Enzym in beliebiger Position um 1, 2 oder 3 Aminosäuren verkürzt oder verlängert sein kann oder wobei eine Aminosäure durch eine andere Aminosäure ersetzt sein kann.

13. Verfahren zur Überführung von Hydroxysäuren in Ketosäuren und umgekehrt unter Verwendung von D-HicDH gemäß Anspruch 12, ggf. erhalten mit Hilfe eines E.-coli-Stamms gemäß Anspruch 10.

14. Verfahren nach Anspruch 13 zur Herstellung von D-Hydroxysäuren.

21

Fig. 1

Fig. 2

A

D-HicDH gene

B

EP 0 351 575 A2

Fig. 3

```
  1    GAGCAAACCATCACAATACCGGTGACTTACCATGGACACCGTTGCAAGAAGCCGTCTGGC

 61    AGTATCTGCAAACGATTCCTTATGGAGAGACCCGAACGTACGCACAGGTGGCAGCCGCAG

121    TCGGTCATCCGCACGCCTTCCAAGCAGTCGGCTCTGAGGTCGGGAAGAATCCCGTTATGA

181    TCGCTGTGCCTTGTCATCGCGTCCTGCGCAAAGATGGTGGTTTGGGTGGCTTTCGTGGCG

241    GTTTGCCAATGAAGCGCGACTTGTTGGCACTTGAACAAGGCAGCGGCCAATTTTTTAAAG

301    ATGCTTTTTCACAGAGGCATCTTGTATACGGTGAGTGGACAAATTGGAAAGGAAGTTTAA

              M  K  I  I  A  Y  G  A  R  V  D  E  I  Q  Y  F  K  Q  W
361    CACATGAAGATTATTGCTTACGGTGCTCGCGTTGACGAGATTCAATATTTCAAGCAATGG

        A  K  D  T  G  N  T  L  E  Y  H  T  E  F  L  D  E  N  T  V
421    GCCAAGGATACAGGCAACACACTTGAATACCATACAGAATTTCTCGATGAAAACACCGTT

        E  W  A  K  G  F  D  G  I  N  S  L  Q  T  T  P  Y  A  A  G
481    GAATGGGCTAAAGGGTTTGATGGCATCAATTCATTGCAGACAACGCCATATGCAGCCGGC

        V  F  E  K  M  H  A  Y  G  I  K  F  L  T  I  R  N  V  G  T
541    GTTTTTGAAAAAATGCACGCGTATGGTATCAAGTTCTTGACGATTCGGAATGTGGGTACG

        D  N  I  D  M  T  A  M  K  Q  Y  G  I  R  L  S  N  V  P  A
601    GATAACATTGATATGACTGCCATGAAGCAATACGGCATTCGTTTGAGCAATGTACCGGCT

        Y  S  P  A  A  I  A  E  F  A  L  T  D  T  L  Y  L  L  R  N
661    TATTCGCCAGCAGCGATTGCTGAATTTGCTTTGACCGATACTTTGTACTTGCTACGTAAT

        M  G  K  V  Q  A  Q  L  Q  A  G  D  Y  E  K  A  G  T  F  I
721    ATGGGTAAAGTACAGGCGCAACTACAGGCGGGCGATTATGAAAAAGCGGGCACCTTCATC

        G  K  E  L  G  Q  Q  T  V  G  V  M  G  T  G  H  I  G  Q  V
781    GGTAAGGAACTCGGTCAGCAAACCGTTGGCGTGATGGGCACCGGTCATATTGGACAGGTT

        A  I  K  L  F  K  G  F  G  A  K  V  I  A  Y  D  P  Y  P  M
841    GCTATCAAACTGTTCAAAGGCTTTGGCGCCAAAGTGATTGCTTACGATCCTTATCCAATG

        K  G  D  H  P  D  F  D  Y  V  S  L  E  D  L  F  K  Q  S  D
901    AAGGGCGATCACCCAGATTTTGACTATGTCAGCCTTGAAGACCTCTTTAAGCAAAGTGAT

        V  I  D  L  H  V  P  G  I  E  Q  N  T  H  I  I  N  E  A  A
961    GTCATTGATCTTCATGTTCCTGGGATTGAACAAAATACCCACATTATCAATGAAGCGGCA

        F  N  L  M  K  P  G  A  I  V  I  N  T  A  R  P  N  L  I  D
1021   TTTAATTTGATGAAACCGGGTGCGATTGTGATCAACACGGCTCGGCCAAATCTGATTGAC

        T  Q  A  M  L  S  N  L  K  S  G  K  L  A  G  V  G  I  D  T
1081   ACGCAAGCCATGCTCAGCAATCTTAAGTCTGGCAAGTTGGCCGGTGTCGGGATTGACACC

        Y  E  Y  E  T  E  D  L  L  N  L  A  K  H  G  S  F  K  D  P
1141   TATGAATACGAAACCGAGGACTTGTTGAATCTCGCCAAGCACGGCAGCTTCAAGGATCCG

        L  W  D  E  L  L  G  M  P  N  V  V  L  S  P  H  I  A  Y  Y
1201   TTGTGGGACGAGCTGTTGGGGATGCCAAATGTTGTCCTCAGCCCGCACATTGCCTACTAC

        T  E  T  A  V  H  N  M  V  Y  F  S  L  Q  H  L  V  D  F  L
1261   ACCGAGACGGCTGTGCATAATATGGTTTACTTCTCACTACAACATCTCGTTGATTTCTTG

        T  K  F  K  P  A  R  K  L  L  V  Q  Q  V  V  N  *
1321   ACCAAATTCAAACCAGCACGGAAGTTACTGGTCCAGCAAGTAGTCAACTGAATAGCAAAA

1381   CGTGCCCTGCCTATTGACGAATACGTTAATGGCAGAGCACGTTTTTTTAATATTGGGATTG

1441   CTTGCTTAAAAAAATCGAATTGCCACGAATTGCCATGATATAAACAAACAAGGCAGCGCCA

1501   TTGACGAACAAGGAGACGGCAATGATTGAAAAACGAATGGTGTACGGTCTGGCGCCTATG

1561   TCAGGATCATAAAAGCGTGAACGGCGTTTG
```

Fig. 4

```
                    A U
                   A   A
                   G ≡ C
                   C ≡ G
                   A = U
                   G • U
                   U = A
                   U = A
                   A = U
                 U
                   C ≡ G
                   C ≡ G
                   G ≡ C
                   U = A
                   C ≡ G
                 C           A
                   C ≡ G
                   G ≡ C
                   G = A
                   G ≡ C
                   C ≡ G
                   A = U
                   A = U
                   A = U
              G C A = U U U
            A                  A
          U                      A
         A                        U
         A                        A
    STOP  G                       U
         U                        U
          C                      U
           A A  C ≡ G   G    G
                U = A
                G • U
                A = U
                U • G
                G ≡ C
                A = U
                A = U
                C ≡ G
                G ≡ C
                A = U
    A C U G G U C C     U A A A A A A U
```

Fig. 5

(a)  ATGAAGATTATTGCTTACGGTGCTCGCGTTGACGAGATTCAATATTTCAAGCAATGGGCCAAGGA
     ***** ******** ** ***** ** *****  * *********** **  ****** ** **
(b)  ATGAAAATTATTGCGTATGGTGCGCGTGTTGATTATATTCAATATTTTAAAGAATGGGCGAAAGA

Fig. 6

Fig. 7

EP 0 351 575 A2